# EUROPEAN PATENT APPLICATION

(11) **EP 2 123 303 A1**
(43) Date of publication of application: **25.11.2009**
(21) Application number: 08711588.7
(22) Date of filing: 19.02.2008
(51) Int. Cl.: A61K 45/00, A61K 31/7105, A61K 48/00, A61P 1/16, A61P 3/00, A61P 3/04, A61P 3/06, A61P 3/10, A61P 9/04, A61P 9/10, A61P 9/12, A61P 13/12, A61P 25/00, A61P 27/02, A61P 43/00, G01N 33/15, G01N 33/50

(54) **AMELIORATING AGENT FOR INSULIN RESISTANCE**

(30) Priority: 20.02.2007 JP 2007039947
(71) Applicant: Takeda Pharmaceutical Company Limited, Osaka 541-0045 (JP)
(72) Inventor: KIZAWA, Hideki, Tsukuba-shi Ibaraki 300-4293 (JP); FUKUSUMI, Shoji, Osaka-shi Osaka 532-8686 (JP)
(74) Representative: Jones, Nicholas Andrew
(86) International application number: PCT/JP2008/052767
(87) International publication number: WO 2008/102777

(57) **Abstract**

The present invention provides an insulin sensitizer and a prophylactic/therapeutic agent for diseases involved by sugar metabolic abnormality, comprising a substance that inhibits the expression or activity of CPSF5 protein and/or a substance that inhibits the expression or activity of CPSF6 protein. Provided as the substances are (a) an antisense nucleic acid against a nucleic acid that encodes CPSF5 (or CPSF6), (b) an siRNA against an RNA that encodes CPSF5 (or CPSF6), (c) a nucleic acid capable of producing an siRNA against an RNA that encodes CPSF5 (or CPSF6), and the like. Also provided is a screening method for an insulin resistance ameliorating substance using a cell that produces CPSF5 and/or CPSF6.

## Description

### [Technical Field]

The present invention relates to an insulin sensitizer, a prophylactic/therapeutic agent for diabetes, and screening therefor.

### [Background of the Invention]

Insulin resistance is a pathologic condition characterized by decreased insulin sensitivity in the liver, skeletal muscles, and adipose; particularly in type II diabetes, in addition to insulin secretion insufficiency, insulin resistance is a major etiology involved in the onset and progression of diabetes. Generally, since many of diabetic patients with obesity have insulin resistance, insulin resistance is thought to be profoundly associated with obesity. Furthermore, it is known that insulin resistance is also seen not only in diabetes, but also in diseases caused by lipid metabolic abnormalities, such as arteriosclerosis (non-patent document 1).

In diabetic patients, accentuated sugar release in the liver and decreased sugar uptake in the liver are observed, both being of paramount importance in the formation of hyperglycemic state. Factors that determine hepatic sugar release are divided into impaired control of the glycogen decomposition and synthesis system and hyperfunction of the gluconeogenesis system; in particular, abnormalities in the mechanism for the gluconeogenesis system in diabetes are attracting attention. In the regulation of hepatic gluconeogenesis, phosphoenolpyruvate carboxykinase (PEPCK), glucose-6-phosphatase (G6Pase) and the like work as rate-limiting enzymes. It is known that when these enzymes are allowed to be overexpressed in the mouse liver, insulin resistance and impaired glucose tolerance are caused, and that in the livers of various animal models of diabetes, the expression of these enzymes is accentuated (non-patent document 2).

Suppressing the expression of these enzymes in the liver is expected to lead to diabetic treatment in the future (non-patent document 3). Specifically, regarding hepatic insulin resistance, as factors responsible for the transcriptional regulation of these enzymes, forkhead box O1 (Foxo1), peroxisome proliferator-activated receptor gamma coactivator 1 alpha (PGC-1α) have been reported. PGC-1α serves to activate the transcription in the genetic expression regulatory mechanism for the representative gluconeogenesis enzyme PEPCK. Foxo1 positively regulates the transcription of both PGC-1α and PEPCK. Foxo1 is negatively controlled by insulin. Therefore, because insulin action reduces the activities of both Foxo1 and PGC-1α, gluconeogenesis is suppressed (non-patent document 3). From this fact, these transcriptional regulatory factors are thought to be drug discovery targets for suppression of hepatic insulin resistance (non-patent documents 3 and 4).

There are various processes to allow genetic information on DNA to be expressed as proteins. The mRNA precursors produced as a result of transcription from DNA undergo various processings, are transported to cytoplasm, and work as templates for protein synthesis. In addition to mRNA, other RNAs such as tRNA, Uridine-rich small nuclear RNA (Usn RNA), and micro RNA (miRNA) exhibit essential functions. RNAs play the central role in the gene expression process and perform complex and exquisite regulation of gene expression, thus producing the expressional diversity. If an irregularity occurs in this regulatory mechanism, a disease emerges at the individual level. In addition to transcriptional regulation, abnormalities in these gene expression processes have been identified to date as causes of a large number of diseases. For example, mRNA splicing abnormalities include, for example, familial hypercholesterolemia (LDL-R splicing abnormality) and thalassemia (β-globin splicing abnormality); abnormalities of 3' end processing and RNA transportation include, for example, oculopharyngeal dystrophy (GCG repeat amplification of PABP2) and thrombotic predisposition (prothrombin polyA mutation); RNA editing abnormalities include, for example, Alzheimer's disease and Huntington's disease (Editing abnormalities of GluR2) (non-patent document 5). Hence, it has been evident that in addition to the function of transcriptional regulation, RNAs are associated with diseases.

CPSF5 and CPSF6 are subunits that constitute the Cleavage factor I, mammal (CFIm), an enzyme complex that catalyzes the processing of mRNA precursor 3' end. CPSF5 and CPSF6 are genes included in a group of genes associated with polyA addition to the 3' end of mRNA, and are necessary for the promotion of cleavage at the 3' end. It has also been reported that when a polyA addition signal is present at the 3' end of mRNA, CPSF5 is required for determination of the cleavage site (non-patent document 6). Furthermore, CPSF5 and CPSF6 have recently been reported to be also associated with splicing (non-patent documents 7 and 8).
[Non-patent document 1] Saltiel, A.R., Cell, Vol.104, pp.517-529, 2001
[Non-patent document 2] Friedman, J.E. et al., J. Biol. Chem., Vol.272, pp.31475-31481, 1997
[Non-patent document 3] Samuel, V.T. et al., Diabetes, Vol.55, pp.2042-2050, 2006
[Non-patent document 4] Puigserver, P. et al., Nature, Vol.423, pp.550-555, 2003
[Non-patent document 5] Stoilov, P. et al., DNA Cell Biol., Vol.21, pp.803-818, 2002
[Non-patent document 6] Krainer, A.R. ed., "Eukaryotic mRNA Processing" IRL (Oxford University) Press, 1997
[Non-patent document 7] Millevoi, S. et al., EMBO J., Vol.25, pp.4854-4864, 2006
[Non-patent document 8] Kubo, T. et al., Nucleic Acids Res., Vol.34, pp.6264-6271, 2006

### [Disclosure of the Invention]

### [Problems to Be Solved by the Invention]

There is demand for a safe, effective therapeutic drug for diabetes that ameliorates insulin resistance.

### [Means of Solving the Problems]

RNA interference is a technique wherein double-stranded RNA specifically decomposes mRNA via the RNA-induced silencing complex (RISC) effect to regulate the translation or transcription, and it can suppress genes with sequence specificity; research and development activities are ongoing with its future application to pharmaceuticals in mind. This technique also allows genes to be knocked down easily, and therefore can be used for experiments of the loss of gene functions at the laboratory level. Furthermore, considering systemic administration in vivo, the liver is an organ to which nucleic acids are likely to reach; as a step that follows exploratory studies for a drug discovery target by means of nucleic acids such as short interfering RNA (siRNA) and antisense oligonucleotides, administration of modified nucleic acids in combination with a simple nucleic acid delivery system for pharmaceutical applications and the like is highly feasible in terms of organ specificity.
Based on these findings, the present inventors, in an attempt to obtain a means for solving the above-described problems, knocked down various genes expected to be related to RNA functions using siRNAs, and searched for genes involved in insulin resistance in the liver with hepatic gluconeogenesis as an index. As a result, the inventors found that siRNAs against CPSF5 (cleavage and polyadenylation specificity factor 5) and CPSF6 (cleavage and polyadenylation specificity factor 6) possess hepatic insulin resistance ameliorating action.
The present inventors conducted further investigations based on these findings, and have developed the present invention.

Accordingly, the present invention provides:
[1] an insulin sensitizer comprising a substance inhibiting expression or activity of a protein comprising an amino acid sequence which is the same or substantially the same as the amino acid sequence shown by SEQ ID NO: 2, and/or a substance inhibiting expression or activity of a protein comprising an amino acid sequence which is the same or substantially the same as the amino acid sequence shown by SEQ ID NO: 4;
[2] the sensitizer of the above-mentioned [1], wherein the substance inhibiting expression of a protein comprising an amino acid sequence which is the same or substantially the same as the amino acid sequence shown by SEQ ID NO: 2, and/or the substance inhibiting expression of a protein comprising an amino acid sequence which is the same or substantially the same as the amino acid sequence shown by SEQ ID NO: 4 are/is any of the following (a) to (c):
   (a) an antisense nucleic acid to a nucleic acid encoding each protein
   (b) siRNA to RNA encoding each protein
   (c) a nucleic acid capable of producing siRNA to RNA encoding each protein;
[3] the sensitizer of the above-mentioned [1] having a gluconeogenesis inhibitory action;
[4] the sensitizer of the above-mentioned [1], which is an agent for the prophylaxis or treatment of a disease involving a glucose metabolism disorder;
[5] a method of improving insulin resistance in an animal, comprising administering, to the animal, (an) effective amount(s) of a substance inhibiting expression or activity of a protein comprising an amino acid sequence which is the same or substantially the same as the amino acid sequence shown by SEQ ID NO: 2, and/or a substance inhibiting expression or activity of a protein comprising an amino acid sequence which is the same or substantially the same as the amino acid sequence shown by SEQ ID NO: 4;
[6] use of a substance inhibiting expression of a protein comprising an amino acid sequence which is the same or substantially the same as the amino acid sequence shown by SEQ ID NO: 2, and/or a substance inhibiting expression of a protein comprising an amino acid sequence which is the same or substantially the same as the amino acid sequence shown by SEQ ID NO: 4, for the production of an insulin sensitizer;
[7] a method of screening for an insulin sensitizing substance, comprising contacting cells producing the following (a) and/or (b) :
   (a) a protein comprising an amino acid sequence which is the same or substantially the same as the amino acid sequence shown by SEQ ID NO: 2 or a partial peptide thereof
   (b) a protein comprising an amino acid sequence which is the same or substantially the same as the amino acid sequence shown by SEQ ID NO: 4 or a partial peptide thereof
   with a test compound, and measuring an expression level or activity of the protein of said (a) or a partial peptide thereof and/or the protein of said (b) or a partial peptide thereof;
[8] the method of the above-mentioned [7], wherein the insulin sensitizing substance has a gluconeogenesis inhibitory action;
[9] the method of the above-mentioned [7], wherein the insulin sensitizing substance can prevent or treat a disease involving a glucose metabolism disorder;
and the like.

### [Effect of the Invention]

Because a substance that inhibits the expression or activity of CPSF5 and/or CPSF6 suppresses insulin-stimulated gluconeogenesis on one hand and does not influence dexamethasone (Dex)/8-(4-CHLOROPHENYLTHIO)-ADENOSINE 3':5'-CYCLIC MONOPHOSPHATE SODIUM SALT (8CPT)-stimulated sugar production on the other hand, the substance exhibits the remarkable advantage of ameliorating insulin resistance without causing toxic signs such as lactate acidosis, and can be used as a safe and effective anti-diabetic drug and the like.

### [Brief Description of the Drawings]

[FIG. 1] A graphic representation showing the suppressive actions of H4IIE-C3-No.75 strain (A) and 76 strain (B) on insulin-stimulated sugar production.
[FIG. 2] A tabulation showing the target RNA-related factors contained in a library of siRNAs.
[FIG. 3] A graphic representation showing results of evaluations of sugar production by siRNAs against CPSF5 and CPSF6 (A and B) and Taqman analyses of mRNA knock-down (C and D). In the graphs, NC indicates a negative control (no siRNA introduced).

### [Detailed Description of the Invention]

CPSF5 in the present invention is a protein comprising the same or substantially the same amino acid sequence as the amino acid sequence shown by SEQ ID NO:2. CPSF6 in the present invention is a protein comprising the same or substantially the same amino acid sequence as the amino acid sequence shown by SEQ ID NO:4. Herein, proteins and peptides are described with the left end indicating the N-terminus (amino terminus) and the right end indicating the C-terminus (carboxyl terminus), according to the common practice of peptide designation.
The CPSF5 and CPSF6 proteins may be ones isolated/purified from cells [e.g., hepatocyte, splenocyte, nerve cell, glial cell, pancreatic β cell, myelocyte, mesangial cell, Langerhans' cell, epidermal cell, epithelial cell, goblet cell, endothelial cell, smooth muscle cell, fibroblast, fibrocyte, myocyte, adipocyte, immune cell (e.g., macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell, interstitial cell, or a corresponding precursor cell, stem cell or cancer cell thereof, and the like] of humans or other warm-blooded animals (for example, guinea pigs, rats, mice, chicken, rabbits, dogs, pigs, sheep, bovines, monkeys and the like) or any tissues where such cells are present [for example, brain or each part of brain (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscles (e.g., smooth muscle, skeletal muscle), lung, gastrointestinal tract (e.g., large intestine, small intestine), blood vessel, heart, thymus, spleen, submandibular gland, peripheral blood, prostate, testis, ovary, placenta, uterus, bone, joint, adipose tissue (e.g., white adipose tissue, brown adipose tissue) and the like] by a method of protein separation and purification known per se.

As substantially the same amino acid sequence as that shown by SEQ ID NO:2 (or SEQ ID NO:4), an amino acid sequence having a homology of about 50% or more, preferably about 60% or more, more preferably about 70% or more, still more preferably about 80% or more, particularly preferably about 90% or more, most preferably about 95% or more, to the amino acid sequence shown by SEQ ID NO:2 or SEQ ID NO:4 and the like can be mentioned. As used herein, "homology" means the proportion (%) of the same and similar amino acid residues to all overlapping amino acid residues in the optimal alignment where two amino acid sequences are aligned using a mathematic algorithm known in the relevant technical field (preferably, the algorithm is such that a gap can be introduced into one or both of the sequences for the optimal alignment). "A similar amino acid" means an amino acid having similar physiochemical properties; as examples, amino acids classified under the same group, such as aromatic amino acids (Phe, Trp, Tyr), aliphatic amino acids (Ala, Leu, Ile, Val), polar amino acids (Gln, Asn), basic amino acids (Lys, Arg, His), acidic amino acids (Glu, Asp), amino acids having a hydroxy group (Ser, Thr), and amino acids having a small side chain (Gly, Ala, Ser, Thr, Met), can be mentioned. Substitution by such similar amino acids is expected to give no change in the phenotype of protein (i.e., constitutive amino acid substitution). Specific examples of conservative amino acid substitution are known in the relevant technical field and described in various pieces of the literature (see, for example, Bowie et al., Science, 247: 1306-1310 (1990)).

Amino acid sequence homology herein can be calculated using the homology calculation algorithm NCBI BLAST (National Center for Biotechnology Information Basic Local Alignment Search Tool) under the following conditions (expectancy=10; gap allowed; matrix=BLOSUM62; filtering=OFF). Algorithms to determine the homology of an amino acid sequence include, for example, the algorithm described in Karlin et al., Proc. Natl. Acad. Sci. USA, 90: 5873-5877 (1993) [the algorithm is incorporated in the NBLAST and XBLAST programs (version 2.0) (Altschul et al., Nucleic Acids Res., 25: 3389-3402 (1997))], the algorithm described in Needleman et al., J. Mol. Biol., 48: 444-453 (1970) [the algorithm is incorporated in the GAP program in the GCG software package], the algorithm described in Myers and Miller, CABIOS, 4: 11-17 (1988) [the algorithm is incorporated in the ALIGN program (version 2.0), which is part of the CGC sequence alignment software package], the algorithm described in Pearson et al., Proc. Natl. Acad. Sci. USA, 85: 2444-2448 (1988) [the algorithm is incorporated in the FASTA program in the GCG software package] and the like, and they can also be used preferably.

More preferably, substantially the same amino acid sequence as the amino acid sequence shown by SEQ ID NO:2 (or SEQ ID NO:4) is an amino acid sequence having a homology of about 50% or more, preferably about 60% or more, more preferably about 70% or more, still more preferably about 80% or more, particularly preferably about 90% or more, and most preferably about 95% or more, to the amino acid sequence shown by SEQ ID NO:2 (or SEQ ID NO:4).

"A protein comprising the same or substantially the same amino acid sequence as the amino acid sequence shown by SEQ ID NO:2 (or SEQ ID NO:4)" is a protein comprising substantially the same amino acid sequence as the amino acid sequence shown by SEQ ID NO:2 (or SEQ ID NO:4), and possessing substantially the same quality of activity as a protein consisting of the amino acid sequence shown by SEQ ID NO:2 (or SEQ ID NO:4).
Here, "activity" is mRNA precursor 3' end processing activity (cleavage factor Iₘ (CFIₘ) activity). "Substantially the same quality" means that the activities are qualitatively, for example, physiologically or pharmacologically, equivalent to each other. Therefore, it is preferable that the CFIₘ activities be equivalent to each other, but the quantitative factors of these activities, such as the extent of activity (e.g., about 0.01 to about 100 times, preferably about 0.1 to about 10 times, more preferably about 0.5 to 2 times) and the molecular weight of the protein, may be different.
A measurement of CFIₘ activity can be performed in accordance with a method known per se, for example, a method described in Ruegsegger et al. (Mol. Cell., Vol.1, pp.243-253, 1998).

Examples of the CPSF5 in the present invention also include what are called muteins of proteins comprising (i) an amino acid sequence having 1 or 2 or more (for example, about 1 to 50, preferably about 1 to 30, more preferably about 1 to 10, still more preferably several (1 to 5, 4, 3 or 2)) amino acids deleted from the amino acid sequence shown by SEQ ID NO:2, (ii) an amino acid sequence having 1 or 2 or more (for example, about 1 to 50, preferably about 1 to 30, more preferably about 1 to 10, still more preferably several (1 to 5, 4, 3 or 2)) amino acids added to the amino acid sequence shown by SEQ ID NO:2, (iii) an amino acid sequence having 1 or 2 or more (for example, about 1 to 50, preferably about 1 to 30, more preferably about 1 to 10, still more preferably several (1 to 5, 4, 3 or 2)) amino acids inserted in the amino acid sequence shown by SEQ ID NO:2, (iv) an amino acid sequence having 1 or 2 or more (for example, about 1 to 50, preferably about 1 to 30, more preferably about 1 to 10, still more preferably several (1 to 5, 4, 3 or 2)) amino acids substituted by other amino acids in the amino acid sequence shown by SEQ ID NO:2, or (v) an amino acid sequence comprising a combination thereof. Likewise, examples of the CPSF6 in the present invention also include what are called muteins of proteins comprising (i) an amino acid sequence having 1 or 2 or more (for example, about 1 to 100, preferably about 1 to 50, more preferably about 1 to 10, still more preferably several (1 to 5, 4, 3 or 2)) amino acids deleted from the amino acid sequence shown by SEQ ID NO:4, (ii) an amino acid sequence having 1 or 2 or more (for example, about 1 to 100, preferably about 1 to 50, more preferably about 1 to 10, still more preferably several (1 to 5, 4, 3 or 2)) amino acids added to the amino acid sequence shown by SEQ ID NO:4, (iii) an amino acid sequence having 1 or 2 or more (for example, about 1 to 100, preferably about 1 to 50, more preferably about 1 to 10, still more preferably several (1 to 5, 4, 3 or 2)) amino acids inserted in the amino acid sequence shown by SEQ ID NO:4, (iv) an amino acid sequence having 1 or 2 or more (for example, about 1 to 100, preferably about 1 to 50, more preferably about 1 to 10, still more preferably several (1 to 5, 4, 3 or 2)) amino acids substituted by other amino acids in the amino acid sequence shown by SEQ ID NO:4, or (v) an amino acid sequence comprising a combination thereof
When an amino acid sequence is inserted, deleted or substituted as described above, the position of the insertion, deletion or substitution is not particularly limited, as far as the mRNA precursor 3' end processing activity (CFIₘ activity) is retained.

As examples of preferred CPSF5 proteins, for example, human CPSF5, which consists of the amino acid sequence shown by SEQ ID NO:2 (RefSeq Accession No. NP_008937.1), or homologues thereof in other mammals (for example, mouse homologue registered with GenBank under RefSeq Accession No. NP_080899.1), and naturally occurring allelic variants thereof and the like can be mentioned. As examples of preferred CPSF6 proteins, for example, human CPSF6, which consists of the amino acid sequence shown by SEQ ID NO:4 (RefSeq Accession No. NP_008938.1), or homologues thereof in other mammals (for example, mouse homologue registered with GenBank under RefSeq Accession No. NP_001013409.1), and naturally occurring allelic variants thereof and the like can be mentioned.

In the present invention, "a substance that inhibits the expression of CPSF5 (or CPSF6) protein" may be one that acts in any stage at the CPSF5 (or CPSF6) gene transcription level, post-transcriptional regulation level, translation-into-protein level, post-translational modification level and the like. Therefore, examples of a substance that inhibits the expression of CPSF5 (or CPSF6) protein include a substance that inhibits the transcription of the gene, a substance that inhibits the processing of the initial transcription product into mRNA, a substance that inhibits the transportation of mRNA to cytoplasm, a substance that promotes the degradation of mRNA, a substance that inhibits the translation of mRNA into protein, a substance that inhibits the post-translational modification of CPSF5 (or CPSF6) polypeptide and the like. Although any one that acts in any stage can be preferably used, a substance that inhibits the translation of mRNA into protein is preferred in that the production of CPSF5 or CPSF6 protein is directly inhibited.

As a substance capable of specifically inhibiting the translation of the mRNA of CPSF5 or CPSF6 into protein, preferably, a nucleic acid comprising a base sequence complementary or substantially complementary to the base sequence of one of these mRNAs or a portion thereof can be mentioned.
A base sequence substantially complementary to the base sequence of the mRNA of CPSF5 or CPSF6 means a base sequence having a complementarity such that the base sequence is capable of binding to the target sequence for the mRNA to inhibit the translation thereof under physiological conditions in the body of a mammal that is manifesting a pathologic condition of insulin resistance, or is assumed to be at a high risk of contracting insulin resistance in the future; specifically, for example, the base sequence is a base sequence having a homology of about 70% or more, preferably about 80% or more, more preferably about 90% or more, and most preferably about 95% or more, with respect to the overlapping region, to a base sequence completely complementary to the base sequence of the mRNA (i.e., the base sequence of a complementary strand of the mRNA).
Homology of the base sequences in the present specification can be calculated under the following conditions (an expectation value = 10; gaps are allowed; filtering = ON; match score = 1; mismatch score = -3) using a homology scoring algorithm NCBI BLAST (National Center for Biotechnology Information Basic Local Alignment Search Tool). As examples of other algorithms for determination of base sequence homology, the algorithm described in Karlin et al., Proc. Natl. Acad. Sci. USA, 90:5873-5877 (1993) [the algorithm is incorporated in the NBLAST and XBLAST programs (version 2.0) (Altschul et al., Nucleic Acids Res., 25:3389-3402 (1997))], the algorithm described in Needleman et al., J. Mol. Biol., 48:444-453 (1970) [the algorithm is incorporated in the GAP program in the GCG software package], the algorithm described in Myers and Miller, CABIOS, 4:11-17 (1988) [the algorithm is incorporated in the ALIGN program (version 2.0), which is part of the CGC sequence alignment software package], and the algorithm described in Pearson et al., Proc. Natl. Acad. Sci. USA, 85:2444-2448 (1988) [the algorithm is incorporated in the FASTA program in the GCG software package] and the like can be mentioned, and these can also be preferably used in the same way.

More specifically, as a base sequence complementary or substantially complementary to the base sequence of the mRNA of CPSF5, a base sequence complementary or substantially complementary to (a) the base sequence shown by SEQ ID NO:1 or (b) a base sequence that hybridizes with the base sequence under high stringent conditions and encodes a protein having substantially the same quality of activity as a protein consisting of the amino acid sequence shown by SEQ ID NO:2 can be mentioned. As a base sequence complementary or substantially complementary to the base sequence of the mRNA of CPSF6, a base sequence complementary or substantially complementary to (a) the base sequence shown by SEQ ID NO:3 or (b) a base sequence that hybridizes with the base sequence under high stringent conditions and encodes a protein having substantially the same quality of activity as a protein consisting of the amino acid sequence shown by SEQ ID NO:4 can be mentioned. Here, "substantially the same quality of activity" is as defined above.
High stringent conditions refer to, for example, conditions involving a sodium concentration of about 19 to about 40mM, preferably about 19 to about 20mM, and a temperature of about 50 to about 70°C, preferably about 60 to about 65°C. In particular, a preferred case is such that the sodium salt concentration is about 19mM and the temperature is about 65°C.

The mRNA of CPSF5 is preferably the human CPSF5 mRNA, which comprises the base sequence shown by SEQ ID NO:1 (RefSeq Accession No. NM_007006.2), or a homologue thereof in another mammal (for example, mouse homologue registered with GenBank under RefSeq Accession No. NM_026623.3), or a naturally occurring allelic variant thereof. The mRNA of CPSF6 is preferably the human CPSF6 mRNA, which comprises the base sequence shown by SEQ ID NO:3 (RefSeq Accession No. NM_007007.1), or a homologue thereof in another mammal (for example, mouse homologue registered with GenBank under RefSeq Accession No. NM_001013391.1), or a naturally occurring allelic variant thereof.

"A portion of a base sequence complementary or substantially complementary to the base sequence of the mRNA of CPSF5 or CPSF6" is not particularly limited with respect to the length and position thereof, as far as the portion is capable of binding specifically to the mRNA of CPSF5 or CPSF6, and capable of inhibiting the protein translation from the mRNA; in terms of sequence specificity, the portion comprises at least 10 bases or more, preferably about 15 bases or more, and more preferably about 20 bases or more, of a portion complementary or substantially complementary to the target sequence.

Specifically, as a nucleic acid comprising a base sequence complementary or substantially complementary to the base sequence of the mRNA of CPSF5 or CPSF6 or a portion thereof, any one of the following (a) to (c) can be preferably mentioned.
(a) An antisense nucleic acid against the mRNA of CPSF5 or CPSF6
(b) An siRNA against the mRNA of CPSF5 or CPSF6
(c) A nucleic acid capable of producing an siRNA against the mRNA of CPSF5 or CPSF6

### (a) An antisense nucleic acid against the mRNA of CPSF5 or CPSF6

"An antisense nucleic acid against the mRNA of CPSF5 or CPSF6" in the present invention is a nucleic acid comprising a base sequence complementary or substantially complementary to the base sequence of the mRNA or a portion thereof, and having the function of suppressing protein synthesis by binding to the target mRNA while forming a specific and stable double strand therewith.
Examples of the antisense nucleic acid include polydeoxyribonucleotides comprising 2-deoxy-D-ribose, polyribonucleotides comprising D-ribose, other types of polynucleotides being N-glycosides of the purine or pyrimidine base, other polymers having a non-nucleotide backbone (for example, commercially available protein nucleic acids and nucleic acid polymers specific for synthetic sequences) or other polymers comprising a special linkage (provided that the polymers comprise nucleotides having such an alignment that allows base pairing or base attachment, as found in DNA or RNA) and the like. These may be double-stranded DNAs, single-stranded DNAs, double-stranded RNAs, single-stranded RNAs, or DNA:RNA hybrids, and may also be unmodified polynucleotides (or unmodified oligonucleotides); those with known modifications, for example, those with labels known in the art, those with caps, those methylated, those with substitution of one or more naturally occurring nucleotides with their analogues, those with intramolecular modifications of nucleotides such as those with uncharged linkages (for example, methyl phosphonates, phosphotriesters, phosphoramidates, carbamates and the like) and those with charged linkages or sulfur-containing linkages (e.g., phosphorothioates, phosphorodithioates and the like); those having side chain groups such as proteins (nucleases, nuclease inhibitors, toxins, antibodies, signal peptides, poly-L-lysine and the like) or saccharides (e.g., monosaccharides and the like); those with intercalators (e.g., acridine, psoralen and the like); those with chelators (for example, metals, radioactive metals, boron, oxidative metals and the like); those with alkylating agents; or those with modified linkages (for example, α anomeric nucleic acids and the like). Here, "nucleosides", "nucleotides" and "nucleic acids" may include those not only comprising the purine and pyrimidine bases, but also comprising other modified heterocyclic bases. Such modified products may comprise a methylated purine and pyrimidine, an acylated purine and pyrimidine, and another heterocyclic ring. Modified nucleosides and modified nucleotides may have a modification in the sugar moiety thereof; for example, one or more hydroxyl groups may be substituted by halogens, aliphatic groups and the like, or may be converted into functional groups such as ethers and amines.

As stated above, the antisense nucleic acid may be a DNA or RNA, or a DNA/RNA chimera. When the antisense nucleic acid is a DNA, a RNA:DNA hybrid formed by a target RNA and antisense DNA is capable of being recognized by endogenous RNase H to cause selective degradation of the target RNA. Therefore, in the case of an antisense DNA intended to cause degradation by RNase H, the target sequence may be not only a sequence in the mRNA, but also the sequence of an intron region in the initial translation product of the CPSF5 or CPSF6 gene. For example, in the case of humans, the CPSF5 gene and the CPSF6 gene are present in the 16q13 region of chromosome 16 and the 12q15 region of chromosome 12, respectively, so that the intron sequence can be determined by comparing the genomic sequences of these regions and the human CPSF5 cDNA base sequence shown by SEQ ID NO:1 and the human CPSF6 cDNA base sequence shown by SEQ ID NO:3 using a homology search program such as BLAST or FASTA.

The target region for an antisense nucleic acid of the present invention is not particularly limited with respect to the length thereof, as far as the translation into CPSF5 or CPSF6 protein is inhibited as a result of hybridization of the antisense nucleic acid; the target region may be the entire sequence or a partial sequence of the mRNA that encodes the protein, and the length is about 10 bases for the shortest, and the entire sequence of the mRNA or initial transcription product for the longest. Taking into account the issues of the ease of synthesis, antigenicity, and intracellular migration and the like, an oligonucleotide consisting of about 10 to about 40 bases, particularly about 15 to about 30 bases, is preferable, but this is not to be construed as limiting. Specifically, the 5' end hairpin loops, 5' end 6-base-pair repeats, 5' end noncoding regions, translation initiation codons, protein coding regions, ORF translation stop codons, 3' end noncoding regions, 3' end palindrome regions, 3' end hairpin loops and the like of the CPSF5 and CPSF6 genes can be chosen as preferable target regions for the antisense nucleic acid, but these are not to be construed as limiting.

Furthermore, an antisense nucleic acid of the present invention may be one that not only hybridizes with the mRNA or initial transcription product of CPSF5 or CPSF6 to inhibit the translation into protein, but also is capable of binding to these genes, which are double-stranded DNAs, to form a triple strand (triplex) and inhibit the transcription into RNA (anti-gene).

Although the nucleotide molecules that constitute the antisense nucleic acid may be natural-type RNAs or DNAs, the molecules can comprise various chemical modifications in order to increase the stability (chemical and/or to-enzyme) or specific activity (affinity for RNA). For example, to prevent degradation by hydrolylases such as nuclease, the phosphoric acid residue (phosphate) of each nucleotide that constitutes the antisense nucleic acid can be substituted with, for example, a chemically modified phosphoric acid residue such as phosphorothioate (PS), methylphosphonate, or phosphorodithionate. The hydroxyl group at the 2'-position of the sugar (ribose) of each nucleotide may be replaced with -OR (R represents, for example, CH₃(2'-O-Me), CH₂CH₂OCH₃(2'-O-MOE), CH₂CH₂NHC(NH)NH₂, CH₂CONHCH₃, CH₂CH₂CN or the like). Furthermore, a base moiety (pyrimidine, purine) may be chemically modified; for example, introduction of a methyl group or a cationic functional group into the 5-position of the pyrimidine base, substitution of the 2-position carbonyl group with thiocarbonyl and the like can be mentioned.

Regarding the conformation of the sugar moiety of RNA, two types are dominant: C2'-endo (type S) and C3'-endo (type N); in single-stranded RNA, the sugar moiety occurs in an equilibrium of the two types, but when a double strand is formed, the conformation is fixed for the type N. Therefore, BNA (LNA) (Imanishi, T. et al., Chem. Commun., 1653-9, 2002; Jepsen, J.S. et al., Oligonucleotides, 14, 130-46, 2004), or ENA (Morita, K. et al., Nucleosides Nucleotides Nucleic Acids, 22, 1619-21, 2003), an RNA derivative wherein the conformation of the sugar moiety is fixed for the type N by bridging the 2' oxygen and 4' carbon so as to confer strong bindability to the target RNA, can also be preferably used.

An antisense oligonucleotide of the present invention can be prepared by determining the target sequence for the mRNA or initial transcription product on the basis of the cDNA sequence or genomic DNA sequence of CPSF5 or CPSF6, and synthesizing a sequence complementary thereto using a commercially available automated DNA/RNA synthesizer (Applied Biosystems, Beckman and the like). All antisense nucleic acids comprising the aforementioned various modifications can be chemically synthesized by techniques known per se.

### (b) siRNA against mRNA of CPSF5 or CPSF6

Herein, a double-stranded RNA consisting of an oligo-RNA complementary to the mRNA of CPSF5 or CPSF6 and a complementary chain thereof, what is called an siRNA, is also defined as being included in nucleic acids comprising a base sequence complementary or substantially complementary to the base sequence of the mRNA of CPSF5 or CPSF6 or a portion thereof. It had been known that so-called RNA interference (RNAi), which is a phenomenon wherein if short double-stranded RNA is introduced into a cell, mRNAs complementary to the RNA are degraded, occurs in nematodes, insects, plants and the like; since this phenomenon was confirmed to also occur widely in animal cells [Nature, 411(6836): 494-498 (2001)], RNAi has been widely utilized as an alternative technique to ribozymes. An siRNA can be designed as appropriate on the basis of base sequence information on the target mRNA using commercially available software (e.g., RNAi Designer; Invitrogen). Specifically, examples of preferable siRNAs of the present invention include, but are not limited to, siRNAs used in Examples described below and the like.

Ribonucleoside molecules constituting an siRNA may also undergo the same modifications as with the above-described antisense nucleic acids in order to increase the stability, specific activity and the like. However, in the case of an siRNA, if all ribonucleoside molecules in the natural type RNA are substituted by the modified form, the RNAi activity is sometimes lost, so that it is necessary that the minimum number of modified nucleosides be introduced to allow the RISC complex to function.

An siRNA can be prepared by synthesizing a sense chain and antisense chain of a target sequence on the mRNA using an automated DNA/RNA synthesizer, respectively, and denaturing the chains in an appropriate annealing buffer solution at about 90 to about 95°C for about 1 minute, and thereafter annealing the chains at about 30 to about 70°C for about 1 to about 8 hours. An siRNA can also be prepared by synthesizing a short hairpin RNA (shRNA) serving as an siRNA precursor, and cleaving this using a dicer.

### (c) Nucleic acids capable of producing siRNA against mRNA of CPSF5 or CPSF6

Herein, a nucleic acid designed to be capable of producing the above-described siRNA against the mRNA of CPSF5 or CPSF6 in a living organism is also defined as being included in nucleic acids comprising a base sequence complementary or substantially complementary to the base sequence of the mRNA of CPSF5 or CPSF6 or a portion thereof. As such nucleic acids, the aforementioned shRNA, expression vectors constructed to express the sHRNA and the like can be mentioned. An shRNA can be prepared by designing an oligo-RNA comprising a base sequence prepared by joining a sense chain and antisense chain of a target sequence on mRNA via a spacer sequence having a length allowing it to form an appropriate loop structure (for example, about 15 to 25 bases) inserted therebetween, and synthesizing this using an automated DNA/RNA synthesizer. An expression vector comprising an shRNA expression cassette can be prepared by preparing a double-stranded DNA that encodes the above-described shRNA by a conventional method, and thereafter inserting the DNA into an appropriate expression vector. As the shRNA expression vector, one having a Pol III system promoter such as U6 or H1 can be used. In this case, an shRNA transcribed in an animal cell incorporating the expression vector forms a loop by itself, and is thereafter processed by an endogenous enzyme dicer and the like, whereby a mature siRNA is formed.

As another preferred example of a nucleic acid comprising a base sequence complementary or substantially complementary to the base sequence of the mRNA of CPSF5 or CPSF6 or a portion thereof, a ribozyme capable of specifically cleaving the mRNA in the coding region can be mentioned. Although "ribozyme", in the narrow sense, refers to an RNA possessing enzymatic activity to cleave nucleic acids, the term is used herein as a concept encompassing any DNA possessing sequence-specific nucleic acid cleavage activity. The most versatile ribozyme is self-splicing RNA, which is found in infectious RNAs such as viroid and virusoid, and is known in the hammerhead type, hairpin type and the like. The hammerhead type exhibits enzyme activity with about 40 bases, and it is possible to specifically cleave only a target mRNA by rendering several bases at both ends adjacent to the hammerhead structure portion (about 10 bases in total) complementary to the desired cleavage site of mRNA. Because this type of ribozyme has RNA as the only substrate, the same has a further advantage that genomic DNA is never targeted. When the CPSF5 or CPSF6 mRNA has a double strand structure by itself, the target sequence can be made to be single stranded by using a hybrid ribozyme ligated with an RNA motif derived from a virus nucleic acid capable of binding specifically to RNA helicase [Proc. Natl. Acad. Sci. USA, 98(10): 5572-5577 (2001)]. Furthermore, when ribozyme is used in the form of an expression vector comprising the DNA that encodes the same, the ribozyme may be a hybrid ribozyme further coupled with a sequence of altered tRNA to promote the transfer of the transcription product to cytoplasm [Nucleic Acids Res., 29(13): 2780-2788 (2001)].

A nucleic acid comprising a base sequence complementary or substantially complementary to the base sequence of the mRNA of CPSF5 or CPSF6 or a portion thereof can be supplied in a special form such as liposomes or microspheres, or applied to gene therapy, or administered in a form added to something. Nucleic acids used in such attached forms include polycations that act to neutralize the charge of phosphate backbone, such as polylysines, and hydrophobic ones such as lipids (e.g., phospholipids, cholesterols and the like) that enhance the interaction with cell membrane or increase nucleic acid uptake. Lipids preferred for addition are cholesterols and derivatives thereof (e.g., cholesteryl chloroformate, cholic acid and the like). These moieties may be attached to the 3' end or 5' end of a nucleic acid, and can also be attached via a base, sugar, or intramolecular nucleoside linkage. Other groups may be capping groups placed specifically at the 3' end or 5' end of the nucleic acid to prevent degradation by nucleases such as exonuclease and RNase. Such capping groups include, but are not limited to, hydroxyl protecting groups known in the art, including glycols such as polyethylene glycol and tetraethylene glycol.

The inhibitory activities of these nucleic acids on the expression of CPSF5 (or CPSF6) protein can be examined using a transformant incorporating the CPSF5 (or CPSF6) gene, an in vivo and in vitro expression system for the CPSF5 (or CPSF6) gene, or an in vivo or in vitro translation system for the CPSF5 (or CPSF6) protein.

A substance that inhibits the expression of CPSF5 (or CPSF6) protein in the present invention is not limited to the above-described nucleic acids comprising a base sequence complementary or substantially complementary to the base sequence of the mRNA of CPSF5 or CPSF6 or a portion thereof; as far as the substance directly or indirectly inhibits the production of CPSF5 (or CPSF6) protein, it may be another substance such as a low-molecular compound. Such a substance can be acquired by, for example, the screening method of the present invention described below.

In the present invention, "a substance that inhibits the activity of CPSF5 (or CPSF6) protein" may be any one that prevents CPSF5 (or CPSF6) protein once produced functionally from exhibiting mRNA precursor 3' end processing activity (CFIₘ activity); for example, substances that inhibit the formation of a complex of CPSF5 and CPSF6, substances that inhibit the mRNA bindability of CPSF5, CPSF6 or the complex, substances that inhibit the nuclear migration of CPSF5 or CPSF6 and the like can be mentioned.

Specifically, as an example of a substance that inhibits the activity of CPSF5 (or CPSF6) protein, an antibody against CPSF5 or CPSF6 protein can be mentioned. The antibody may be a polyclonal antibody or a monoclonal antibody. These antibodies can be produced according to a method of antibody or antiserum production known per se. The isotype of the antibody is not particularly limited, and is preferably IgG, IgM or IgA, particularly preferably IgG. The antibody is not particularly limited, as far as it has at least a complementarity determining region (CDR) for specifically recognizing and binding to a target antigen, and the antibody may be, in addition to a complete antibody molecule, for example, a fragment such as Fab, Fab', or F(ab')₂, a conjugate molecule prepared by a gene engineering technique, such as scFv, scFv-Fc, minibody, or diabody, or a derivative thereof modified with a molecule having protein-stabilizing action, such as polyethylene glycol (PEG).
In a preferred embodiment, the antibody against CPSF5 or CPSF6 protein is used as a pharmaceutical for a human recipient, the antibody (preferably monoclonal antibody) is an antibody having a reduced risk of exhibiting antigenicity when administered to humans, specifically a complete human antibody, a humanized antibody, a mouse-human chimera antibody or the like, and particularly preferably a complete human antibody. A humanized antibody and a chimera antibody can be prepared by gene engineering according to a conventional method. Although a complete human antibody can also be produced from a human-human (or mouse) hybridoma, it is desirable, for supplying a large amount of antibody stably and at low cost, that the antibody be produced using a human antibody-producing mouse or the phage display method.

Because CPSF5 and CPSF6 form a CFIₘ complex and play a central role in the processing at the 3' end of mRNA precursor, a substance that inhibits the activity of CPSF5 (or CPSF6) protein is desirably a substance of excellent intracellular migration and nuclear migration. Therefore, a more preferable substance that inhibits the activity of CPSF5 (or CPSF6) protein is a low-molecular compound that complies with Lipinski's Rule. Such a compound can be acquired by, for example, using the screening method of the present invention described below.

Because a substance of the present invention that inhibits the expression or activity of CPSF5 or CPSF6 suppresses insulin-stimulated gluconeogenesis, it is useful in ameliorating the condition of insulin resistance. Additionally, because the substance does not influence Dex/8CPT-stimulated sugar production, it has a further advantage of a low risk of causing toxic signs such as lactate acidosis.
Therefore, a pharmaceutical comprising a substance that inhibits the expression or activity of CPSF5 or CPSF6 can be used as, for example, an insulin sensitizer, a gluconeogenesis inhibitor (in the liver and the like) and the like, as, for example, a prophylactic and/or therapeutic agent for diseases involved by sugar metabolic abnormality, diseases associated with lipid metabolic abnormality, and the like.

### (1) Pharmaceutical containing antisense nucleic acid, siRNA, or precursor nucleic acid thereof

An antisense nucleic acid of the present invention capable of complementarily binding to the transcription product of the CPSF5 or CPSF6 gene to suppress protein translation from the transcription product, an siRNA (or ribozyme) capable of cleaving the transcription product with a homologous (or complementary) base sequence in the transcription product of the CPSF5 or CPSF6 gene as a target, and an shRNA being the precursor of the siRNA and the like (hereinafter, sometimes generically referred to as "a nucleic acid of the present invention") are capable of suppressing the function or action of CPSF5 or CPSF6 protein in vivo, and inhibiting gluconeogenesis action in the liver and the like, and can therefore be used as, for example, insulin sensitizers, gluconeogenesis inhibitors and the like, as, for example, prophylactic/therapeutic agents for diseases associated with sugar metabolism abnormality [e.g., diabetes (preferably type II diabetes), diabetic complications (e.g., neuropathy, nephropathy, retinitis and the like), impaired glucose intolerance, obesity, metabolic syndrome and the like], diseases associated with lipid metabolism abnormality [e.g., arteriosclerosis, hypertension, hyperlipemia (particularly hypertriglyceridemia and the like), fatty liver, non-alcoholic steatohepatitis (NASH), sudden cardiac death, nonfatal myocardial infarction, resting angina pectoris/angina of effort, cardiovascular diseases (e.g., angina pectoris instabilization and the like), cerebrovascular disorders (e.g., cerebral thrombosis, cerebral embolism, cerebral hemorrhage, subarachnoid hemorrhage, transient cerebral ischemic attack and the like) and the like].
A pharmaceutical comprising a nucleic acid of the present invention is of low toxicity, and can be administered as a liquid as it is, or as an appropriate dosage form of pharmaceutical composition, to humans or non-human mammals (e.g., mice, rats, rabbits, sheep, pigs, bovines, cats, dogs, monkeys and the like) orally or parenterally (e.g., intravascular administration, subcutaneous administration and the like).

When a nucleic acid of the present invention is used as the above-described insulin sensitizer, a prophylactic/therapeutic agent for a disease associated with sugar and/or lipid metabolic abnormality and the like, the nucleic acid can be prepared and administered according to a method known per se. That is, a nucleic acid of the present invention, alone or after being functionally inserted into an appropriate expression vector for mammalian cells, such as a retrovirus vector, adenovirus vector, or adenovirus-associated virus vector, can be prepared according to a standard means. The nucleic acid can be administered as it is, or along with an auxiliary for promoting its ingestion, using a gene gun or a catheter such as a hydrogel catheter. Alternatively, the nucleic acid can be prepared as an aerosol and topically administered into the trachea as an inhalant.
Furthermore, for the purpose of improving the disposition, extending the half-life, and increasing the intracellular uptake efficiency, the aforementioned nucleic acid may be prepared as a preparation (injection) alone or with a carrier such as a liposome, and administered intravenously, subcutaneously and the like.

A nucleic acid of the present invention may be administered as it is, or as an appropriate pharmaceutical composition. The pharmaceutical composition used for administration may contain both a nucleic acid of the present invention and a pharmacologically acceptable carrier, diluent or excipient. Such a pharmaceutical composition is supplied in the form of a dosage form suitable for oral or parenteral administration.

As examples of the composition for parenteral administration, injections, suppositories and the like are used; the injections may include dosage forms such as intravenous injections, subcutaneous injections, intracutaneous injections, intramuscular injections and drip infusion injections. Such an injection can be prepared according to a publicly known method. An injection can be prepared by, for example, dissolving, suspending or emulsifying the above-described nucleic acid of the present invention in a sterile aqueous or oily solution in common use for injections. As examples of aqueous solutions for injection, physiological saline, an isotonic solution containing glucose or another auxiliary drug, and the like can be used, which may be used in combination with an appropriate solubilizer, for example, alcohol (e.g., ethanol), polyalcohol (e.g., propylene glycol, polyethylene glycol), non-ionic surfactant [e.g., polysorbate 80, HCO-50 (polyoxyethylene (50 mol) adduct of hydrogenated castor oil)] and the like. As examples of oily solutions, sesame oil, soybean oil and the like can be used, which may be used in combination with benzyl benzoate, benzyl alcohol and the like as solubilizers. The prepared injection solution is preferably filled in an appropriate ampoule. Suppositories used for rectal administration may be prepared by mixing the above-described nucleic acid in an ordinary suppository base.

As the composition for oral administration, solid or liquid dosage forms, specifically tablets (including sugar-coated tables and film-coated tablets), pills, granules, powders, capsules (including soft capsules), syrups, emulsions, suspensions and the like can be mentioned. Such a composition is produced by a publicly known method, and may contain a carrier, diluent or excipient in common use in the field of pharmaceutical making. As examples of the carrier or excipient for tablets, lactose, starch, sucrose, magnesium stearate and the like can be used.

The above-described pharmaceutical composition for parenteral or oral administration is conveniently prepared in a medication unit dosage form suitable for the dosage of the active ingredient. As examples of such a medication unit dosage form, tablets, pills, capsules, injections (ampoules), and suppositories can be mentioned. It is preferable that a nucleic acid of the present invention be contained at, for example, normally 5 to 500 mg, particularly 5 to 100 mg for injections, or 10 to 250 mg for other dosage forms, per medication unit dosage form.

The dose of the above-described pharmaceutical containing a nucleic acid of the present invention varies depending on the subject of administration, target disease, symptoms, route of administration and the like; for example, when the pharmaceutical is used for the treatment/prevention of adult diabetes, it is convenient to administer the nucleic acid of the present invention usually at about 0.01 to 20 mg/kg body weight, preferably about 0.1 to 10 mg/kg body weight, and more preferably about 0.1 to 5 mg/kg body weight, based on a single dose, about 1 to 5 times a day, preferably about 1 to 3 times a day, by intravenous injection. In the case of other modes of parenteral administration and oral administration, similar doses may be administered. In case the symptom is particularly severe, the dose may be increased according to the symptom.

Each of the aforementioned compositions may comprise any other active ingredient that does not produce an unwanted interaction when formulated with a nucleic acid of the present invention.

Furthermore, a nucleic acid of the present invention may be used in combination with other drugs, for example, anti-diabetic drugs such as insulin resistance ameliorating drugs (e.g., thiazolidine derivatives such as troglitazone and pioglitazone and the like), hypoglycemic drugs (e.g., sulfonylurea drugs such as tolbutamide, glyclopyramide, and acetohexamide, sulfonamide drugs such as glymidine and glybuzole, biguanide drugs such as metformin and buformin, and the like), aldose reductase inhibitors (e.g., epalrestat and the like), α-glucosidase inhibitors (e.g., voglibose, acarbose and the like), and somatomedin C preparations (e.g., mecasermin and the like); anti-obesity drugs such as centrally acting anti-obesity drugs (e.g., dexfenfluramine, fenfluramine, phentermine and the like), MCH receptor antagonists (e.g., SB-568849, SNAP-7941 and the like), neuropeptide Y antagonists (e.g., CP-422935 and the like), cannabinoid receptor antagonists (e.g., SR-141716, SR-147778 and the like), ghrelin antagonists, leptin, and β3 agonists, and the like. A nucleic acid of the present invention and the above-described drugs may be administered to the patient at one time or different times.

### (2) Pharmaceuticals containing an antibody against CPSF5 or CPSF6, a low-molecular compound that inhibits the expression or activity of CPSF5 or CPSF6, or the like

Antibodies against CPSF5 or CPSF6 and low-molecular compounds that inhibit the expression or activity of CPSF5 or CPSF6 are capable of inhibiting the production or activity of CPSF5 or CPSF6 protein, or inhibiting the interaction (complex formation) between CPSF5 and CPSF6. Therefore, these substances are capable of suppressing the function or action of CPSF5 or CPSF6 protein in vivo, and inhibiting gluconeogenesis action in the liver and the like, and can be used as, for example, insulin sensitizers, gluconeogenesis inhibitors and the like, as, for example, prophylactic/therapeutic agents for diseases associated with sugar metabolism abnormality [e.g., diabetes (preferably type II diabetes), diabetic complications (e.g., neuropathy, nephropathy, retinitis and the like), impaired glucose intolerance, obesity, metabolic syndrome and the like], diseases associated with lipid metabolism abnormality [e.g., arteriosclerosis, hypertension, hyperlipemia (particularly hypertriglyceridemia and the like), fatty liver, non-alcoholic steatohepatitis (NASH), sudden cardiac death, nonfatal myocardial infarction, resting angina pectoris/angina of effort, cardiovascular diseases (e.g., angina pectoris instabilization and the like), cerebrovascular disorders (e.g., cerebral thrombosis, cerebral embolism, cerebral hemorrhage, subarachnoid hemorrhage, transient cerebral ischemic attack and the like) and the like].
A pharmaceutical comprising the above-described antibody or low-molecular compound is of low toxicity, and can be administered as a liquid as it is, or as an appropriate dosage form of pharmaceutical composition, to humans or mammals (e.g., mice, rats, rabbits, sheep, pigs, bovines, cats, dogs, monkeys and the like) orally or parenterally (e.g., intravascular administration, subcutaneous administration and the like).

The above-described antibody or low-molecular compound may be administered as it is, or as an appropriate pharmaceutical composition. The pharmaceutical composition used for administration may contain both the above-described antibody or low-molecular compound or a salt thereof and a pharmacologically acceptable carrier, diluent or excipient. Such a pharmaceutical composition is provided as a dosage form suitable for oral or parenteral administration.

As examples of the composition for parenteral administration, injections, suppositories and the like are used; the injections may include dosage forms such as intravenous injections, subcutaneous injections, intracutaneous injections, intramuscular injections, and drip infusion injections. Such an injection can be prepared according to a commonly known method. An injection can be prepared by, for example, dissolving, suspending or emulsifying the above-described antibody or low-molecular compound of the present invention or a salt thereof in a sterile aqueous or oily solution in common use for injections. As examples of aqueous solutions for injection, physiological saline, an isotonic solution containing glucose or other auxiliary agent and the like can be used, which may be used in combination with an appropriate solubilizer, for example, an alcohol (e.g., ethanol), a polyalcohol (e.g., propylene glycol, polyethylene glycol), a non-ionic surfactant [e.g., polysorbate 80, HCO-50 (polyoxyethylene (50mol) adduct of hydrogenated castor oil)] and the like. As examples of oily solutions, sesame oil, soybean oil and the like can be used, which may be used in combination with solubilizers such as benzyl benzoate, benzyl alcohol. The injectable preparation prepared is preferably filled in an appropriate ampoule. Suppositories used for rectal administration may be prepared by mixing the above-described antibody or a salt thereof in an ordinary suppository base.

For example, the composition for oral administration includes solid or liquid preparations, specifically, tablets (including sugar-coated tables and film-coated tablets), pills, granules, powdery preparations, capsules (including soft capsules), syrup, emulsions, suspensions, etc. Such a composition is manufactured by publicly known methods and may contain a carrier, a diluent or excipient conventionally used in the field of pharmaceutical preparations. Examples of the carrier or excipient for tablets are lactose, starch, sucrose, magnesium stearate, etc.

Advantageously, the pharmaceutical compositions for parenteral or oral use described above are prepared into pharmaceutical preparations with a unit dose suited to fit a dose of the active ingredients. Such unit dose preparations include, for example, tablets, pills, capsules, injections (ampoules), suppositories, etc. It is preferable that the antibody or low-molecular compound be contained normally at 5 to 500 mg, particularly 5 to 100 mg for injections, or 10 to 250 mg for other dosage forms, per medication unit dosage form.

The dose of the above-described pharmaceutical containing the above-described antibody or low-molecular compound or a salt thereof varies depending on the subject of administration, target disease, symptoms, route of administration and the like; for example, when the pharmaceutical is used for the treatment/prevention of adult diabetes, it is convenient to administer the antibody or low-molecular compound usually at about 0.01 to 20 mg/kg body weight, preferably about 0.1 to 10 mg/kg body weight, and more preferably 0.1 to 5 mg/kg body weight, based on a single dose, about 1 to 5 times a day, preferably about 1 to 3 times a day, by intravenous injection. In the case of other parenteral administrations and oral administration, a dose based thereon can be administered. If the symptom is particularly severe, the dosage may be increased depending on the symptom.

The above-described antibody or low-molecular compound or a salt thereof can be administered as it is, or as an appropriate pharmaceutical composition. The pharmaceutical composition used for the above-described administration contains both the above-described antibody or low-molecular compound or a salt thereof and a pharmacologically acceptable carrier, diluent or excipient. Such a composition is supplied in the form of a dosage form suitable for oral or parenteral administration (e.g., intravascular injection, subcutaneous injection and the like).
Each of the aforementioned compositions may comprise any other active ingredient that does not produce an unwanted interaction when formulated with the above-described antibody or low-molecular compound.
Furthermore, the above-described antibody or low-molecular compound may be used in combination with the same other drugs as those mentioned with respect to pharmaceuticals comprising a nucleic acid of the present invention. The above-described antibody or low-molecular compound and these other drugs may be administered to the patient at one time or different times.

### (3) Screening for candidate compound for pharmaceuticals for diseases

As stated above, when the expression and/or activity of CPSF5 and/or CPSF6 is inhibited, insulin-stimulated gluconeogenesis action in the liver and the like is inhibited. Therefore, a compound that inhibits the expression and/or activity of CPSF5 or CPSF6 protein or a salt thereof can be used as, for example, an insulin sensitizer, a gluconeogenesis inhibitor and the like, as, for example, a prophylactic/therapeutic agent for diseases associated with sugar metabolism abnormality [e.g., diabetes (preferably type II diabetes), diabetic complications (e.g., neuropathy, nephropathy, retinitis and the like), impaired glucose intolerance, obesity, metabolic syndrome and the like], diseases associated with lipid metabolism abnormality [e.g., arteriosclerosis, hypertension, hyperlipemia (particularly hypertriglyceridemia and the like), fatty liver, non-alcoholic steatohepatitis (NASH), sudden cardiac death, nonfatal myocardial infarction, resting angina pectoris/angina of effort, cardiovascular diseases (e.g., angina pectoris instabilization and the like), cerebrovascular disorders (e.g., cerebral thrombosis, cerebral embolism, cerebral hemorrhage, subarachnoid hemorrhage, transient cerebral ischemic attack and the like) and the like].
Therefore, a cell that produces CPSF5 and/or CPSF6 protein or a partial peptide thereof can be used as a tool for screening for a substance possessing insulin resistance ameliorating action, with the expression level and/or activity of the protein (gene) as an index.

When a compound that inhibits the activity of the CPSF5 or CPSF6 or a salt thereof is screened for, the screening method comprises culturing a cell having the capability of producing CPSF5 and/or CPSF6 protein in the presence and absence of a test compound, and comparing the activities of CPSF5 and/or CPSF6 protein under the two conditions.

The cell having the capability of producing CPSF5 and/or CPSF6 protein used in the above-described screening method is not particularly limited, as far as it is a human or other mammalian cell that expresses the protein by nature or a biological sample containing the same (e.g., blood, tissue, organ and the like); preferable examples include cell strains of low sensitivity to insulin (e.g., rat liver-derived cell strains of low sensitivity to insulin that can be established according to a method of an Example below) and the like. In the case of non-human animal blood, tissue, organ and the like, these may be isolated from a living organism and then cultured, or a test compound may be administered to a living organism and then these biological specimens may be isolated after elapse of a given time.
As examples of a cell having the capability of producing CPSF5 and/or CPSF6 protein or a partial peptide thereof, various transformants prepared by gene engineering techniques in public knowledge and common use can be mentioned. As the host, for example, animal cells such as H4IIE-C3 cells, HepG2 cells, HEK293 cells, COS7 cells, and CHO cells are preferably used.

Specifically, such a cell can be prepared by joining a DNA that encodes CPSF5 or a partial peptide thereof (i.e., a DNA comprising the base sequence shown by SEQ ID NO:1 or a base sequence that hybridizes with the former base sequence under high stringent conditions, and encodes a polypeptide that possesses the same quality of activity as a protein consisting of the amino acid sequence shown by SEQ ID NO:2), and/or a DNA that encodes CPSF6 or a partial peptide thereof (i.e., a DNA comprising the base sequence shown by SEQ ID NO:3 or a base sequence that hybridizes with the former base sequence under high stringent conditions, and encodes a polypeptide that possesses the same quality of activity as a protein consisting of the amino acid sequence shown by SEQ ID NO:4), downstream of a promoter in an appropriate expression vector, and introducing the vector into a host animal cell.
A DNA that encodes CPSF5 or a partial peptide thereof and a DNA that encodes CPSF6 or a partial peptide thereof can be synthesized on the basis of the base sequences shown by SEQ ID NO:1 and SEQ ID NO:3 with an appropriate oligonucleotide as a probe or primer, and cloned from a cDNA or cDNA library derived from the aforementioned cell/tissue that produces CPSF5 and CPSF6 using a hybridization method or a PCR method. Hybridization can be performed according to, for example, a method described in Molecular Cloning, 2nd edition (J. Sambrook et al., Cold Spring Harbor Lab. Press, 1989) and the like. When a commercially available library is used, the hybridization can be performed according to the method described in the instruction manual attached to the library.

The base sequence of the DNA can be converted by a method known per se such as the ODA-LA PCR method, the Gapped duplex method, or the Kunkel method, or a method based thereon, using a known kit, for example, Mutan^{™}-super Express Km (Takara Shuzo Co., Ltd.), Mutan^{™}-K (Takara Shuzo Co., Ltd.) and the like.

The cloned DNA can be used as is or, if desired, after digestion with a restriction enzyme or addition of a linker, depending on the purpose of use. The DNA may have ATG as a translation initiation codon at the 5' end thereof, and may have TAA, TGA or TAG as a translation termination codon at the 3' end thereof. These translation initiation and termination codons may be added using an appropriate synthetic DNA adapter.

As the expression vector, animal cell expression plasmids (e.g., pA1-11, pXT1, pRc/CMV, pRc/RSV, pcDNAI/Neo); bacteriophages such as λ phage; animal virus vectors such as retrovirus, vaccinia virus and adenovirus, and the like are used. The promoter may be any promoter that matches well with the host used for gene expression. For example, the SRα promoter, SV40 promoter, LTR promoter, CMV (cytomegalovirus) promoter, RSV (Rous sarcoma virus) promoter, MoMuLV (Moloney mouse leukemia virus) LTR, HSV-TK (herpes simplex virus thymidine kinase) promoter and the like are used. In particular, the CMV promoter, SRα promoter and the like are preferable.
As the expression vector, one optionally comprising an enhancer, a splicing signal, a poly A-addition signal, a selection marker, an SV40 replication origin (hereinafter sometimes abbreviated as SV40 ori) and the like, in addition to the above-described examples, can be used. As examples of the selection marker, the dihydrofolate reductase gene (hereinafter sometimes abbreviated as dhfr, methotrexate (MTX) resistance), the ampicillin resistance gene (hereinafter sometimes abbreviated as amp^{r}), the neomycin resistance gene (hereinafter sometimes abbreviated as neo^{r}, G418 resistance) and the like can be mentioned. In particular, when Chinese hamster cells lacking the dhfr gene are used in combination with the dhfr gene as the selection marker, it is also possible to select the desired gene on a thymidine-free medium.

When both a DNA that encodes CPSF5 and a DNA that encodes CPSF6 are introduced into a host animal cell, these DNAs may be dicistronically inserted onto the same vector, or may be monocistronically inserted using the IRES sequence. Alternatively, these DNAs may be separately inserted into respective expression vectors, and introduced into a host cell by co-transfection.

By transforming a host with the aforementioned expression vector comprising a DNA that encodes CPSF5 and/or CPSF6, a cell that expresses CPSF5 and/or CPSF6 can be produced.
As the host, mammalian cells, for example, HepG2 cells, HEK293 cells, HeLa cells, human FL cells, simian COS-7 cells, simian Vero cells, Chinese hamster ovary cells (hereinafter, abbreviated as CHO cells), CHO cells lacking the dhfr gene (hereinafter, abbreviated as CHO(dhfr⁻) cells), mouse L cells, mouse AtT-20 cells, mouse myeloma cells, rat H4IIE-C3 cells, rat GH3 cells and the like can be used.

Transformation can be performed by the calcium phosphate co-precipitation method, PEG method, electroporation method, microinjection method, lipofection method and the like. For example, the methods described in Saibo Kogaku (Cell Engineering), extra issue 8, Shin Saibo Kogaku Jikken Protocol (New Cell Engineering Experimental Protocol), 263-267 (1995), (published by Shujunsha), and Virology, Vol. 52, 456 (1973), can be used.

Transformant cells obtained as described above, mammalian cells intrinsically having the capability of producing CPSF5 and CPSF6 proteins or a tissue/organ comprising the cells can be cultured in a medium, for example, a minimal essential medium (MEM) containing about 5 to 20% fetal bovine serum [Science, Vol.122, 501(1952)], Dulbecco's modified Eagle medium (DMEM) [Virology, Vol.8, 396(1959)], RPMI 1640 medium [The Journal of the American Medical Association, Vol.199, 519(1967)], 199 medium [Proceeding of the Society for the Biological Medicine, Vol.73, 1(1950)] and the like. The pH of the medium is preferably about 6 to 8. Cultivation is normally performed at about 30 to 40°C, and the culture may be aerated or agitated as necessary.

As examples of test compounds, proteins, peptides, antibodies, non-peptide compounds, synthetic compounds, fermentation products, cell extracts, plant extracts, animal tissue extracts, plasma and the like can be mentioned; these substances may be novel substances or publicly known substances.

Contact of a test compound with the above-described cells can be achieved by, for example, adding the test compound to one of the above-described media or various buffer solutions (for example, HEPES buffer solution, phosphate buffer solution, phosphate-buffered physiological saline, Tris-HCl buffer solution, borate buffer solution, acetate buffer solution and the like), and incubating the cells for a given time. The concentration of the test compound added varies depending on the choice of compound (solubility, toxicity and the like), and can be chosen as appropriate over the range of, for example, about 0.1nM to about 100nM. Incubation time is, for example, about 10 minutes to about 24 hours.

When the cell that produces CPSF5 and CPSF6 proteins is supplied in the form of a non-human mammal individual, the state of the animal individual is not particularly limited, and may be, for example, an animal model of obesity and/or diabetes such as the db/db mouse, ob/ob mouse, KKAy mouse, or Zucker fatty rat. Although the rearing conditions for the animals used are not particularly limited, it is preferable that the animals be reared in an environment of SPF grade or higher. Contact of a test compound and the cell is achieved by administration of the test compound to the animal individual. The route of administration is not particularly limited; for example, intravenous administration, intra-arterial administration, subcutaneous administration, intracutaneous administration, intraperitoneal administration, oral administration, intratracheal administration, rectal administration and the like can be mentioned. The dose is not particularly limited; for example, a single dose can be administered at about 0.5 to 20 mg/kg, 1 to 5 times a day, preferably 1 to 3 times a day, for 1 to 14 days.

A measurement of the CPSF5 and/or CPSF6 activity in the above-described screening method can be performed with, for example, binding to a labeled RNA probe and the like as an index; examples of useful methods for this measurement include, but are not limited to, a method described in Ruegsegger et al. (1998, ibid.) and the like. As test samples for activity measurements, when the cell that produces CPSF5 and/or CPSF6 protein is supplied in the form of a cell culture, tissue, or organ culture, an extract of the culture can be mentioned; when the cell is supplied as a non-human mammal individual comprising the same, an extract of cells, tissue or organ separated from the individual, for example, homogenates of the liver, adipose tissue, skeletal muscle, or tissue section thereof and the like can be mentioned.

For example, in the above-described screening method, if the activity of CPSF5 and/or CPSF6 protein in the presence of a test compound is inhibited by about 20% or more, preferably 30% or more, more preferably about 50% or more, compared with the activity in the absence of the test compound, the test compound or a salt thereof can be selected as a candidate for a substance that inhibits the activity of CPSF5 and/or CPSF6 protein, and hence a substance possessing insulin resistance ameliorating action.

The present invention also provides a screening method for a substance possessing insulin resistance ameliorating action, comprising comparing the expression of CPSF5 and/or CPSF6 protein (gene) in a cell having the capability of producing the protein (gene) in the presence and absence of a test compound. The choices of cell and test compound used in the present method, the mode of contact of the test compound and the cell and the like are the same as those for the above-described method with the activity of CPSF5 and/or CPSF6 protein as an index.

The expression levels of CPSF5 and CPSF6 can be measured at the RNA level by detecting the mRNA of CPSF5 or CPSF6 using a nucleic acid capable of hybridizing with the above-described DNA that encodes CPSF5 or CPSF6 under high stringent conditions, i.e., a nucleic acid with the base sequence shown by SEQ ID NO:1 (SEQ ID NO:3) or a base sequence capable of hybridizing with the base sequence complementary thereto under high stringent conditions (hereinafter, sometimes referred to as "a nucleic acid for detection of the present invention"). Alternatively, the expression levels can also be measured at the protein level by detecting these proteins using the aforementioned antibody against CPSF5 or CPSF6 (hereinafter, sometimes referred to as "an antibody for detection of the present invention").
Therefore, more specifically, the present invention provides:
(a) a screening method for an insulin resistance ameliorating substance, comprising culturing a cell having the capability of producing CPSF5 and/or CPSF6 protein in the presence and absence of a test compound, measuring the amounts of mRNA that encodes the protein under the two conditions using a nucleic acid for detection of the present invention, and comparing the amounts, and
(b) a screening method for an insulin resistance ameliorating substance, comprising culturing a cell having the capability of producing CPSF5 and/or CPSF6 protein in the presence and absence of a test compound, measuring the amounts of the protein under the two conditions using an antibody for detection of the present invention, and comparing the amounts.

For example, a measurement of the amount of mRNA or amount of protein in CPSF5 and/or CPSF6 can be specifically performed as described below.
(i) A drug (for example, insulin, cAMP, glucose and the like) or the like is administered to a normal or disease (for example, diabetes, obesity, hypertension, hyperlipemia and the like) model non-human mammal (for example, mice, rats, rabbits, sheep, pigs, bovines, cats, dogs, monkeys and the like); after elapse of a given time, blood, or a particular organ (for example, liver, adipose tissue, skeletal muscle and the like), or a tissue or cells isolated from an organ are obtained.
   The mRNA of CPSF5 and/or CPSF6 contained in the cells obtained can be quantified by, for example, extracting the mRNA from the cells or the like by an ordinary method, and using, for example, a technique such as RT-PCR, or can also be quantified by a Northern blot analysis known per se. Meanwhile, the amount of CPSF5 and CPSF6 proteins can be quantified using Western blot analysis or the various immunoassay methods described in detail below.
(ii) It is possible to prepare a transformant incorporating a polynucleotide that encodes CPSF5 and/or CPSF6 protein according to the above-described method, and quantify and analyze the CPSF5 and/or CPSF6 protein or mRNA encoding the same, contained in the transformant, in the same manner as the above-described (i).

Screening for a substance that alters the expression level of CPSF5 and/or CPSF6 can be performed by:
(i) administering a test compound to a normal or disease model non-human mammal before a given time in advance of administration of the drug and the like (30 minutes previously to 24 hours previously, preferably 30 minutes previously to 12 hours previously, more preferably 1 hour previously to 6 hours previously) or after a given time (30 minutes later to 3 days later, preferably 1 hour later to 2 days later, more preferably 1 hour later to 24 hours later), or simultaneously with addition of the drug and the like, and quantifying and analyzing the amount of the mRNA that encodes CPSF5 and/or the amount of the mRNA that encodes CPSF6, or the amount of CPSF5 protein and/or the amount of CPSF6 protein, contained in the cells isolated from the animal, after elapse of a given time from administration (30 minutes later to 3 days later, preferably 1 hour later to 2 days later, more preferably 1 hour later to 24 hours later), or by:
(ii) adding a test compound to the medium or buffer solution before beginning culturing the transformant by a conventional method, and incubating the transformant for a given time (1 day later to 7 days later, preferably 1 day later to 3 days later, more preferably 2 days later to 3 days later), and then quantifying and analyzing the amount of the mRNA that encodes CPSF5 and/or the amount of the mRNA that encodes CPSF6, or the amount of CPSF5 protein and/or the amount of CPSF6 protein, contained in the transformant.

A measurement of the amount of CPSF5 and CPSF6 proteins in the above-described screening method (b) can be specifically performed by:
(i) a method wherein an antibody for detection of the present invention, a sample liquid and labeled CPSF5 or CPSF6 protein are competitively reacted, and the labeled protein bound to the antibody is detected, whereby the CPSF5 or CPSF6 protein in the sample liquid is quantified,
(ii) a method wherein a sample liquid, an antibody for detection of the present invention insolubilized on a carrier, and another antibody for detection of the present invention labeled are simultaneously or sequentially reacted, after which the amount (activity) of the labeling agent on the insolubilizing carrier is measured, whereby the CPSF5 or CPSF6 protein in the sample liquid is quantified, and the like.
In the above-described assay (ii), the two kinds of antibodies desirably recognize different portions of CPSF5 or CPSF6 protein. For example, if one antibody is an antibody that recognizes the N ends of the two proteins, the other antibody may be one that reacts with the C ends of the proteins.
Examples of the labeling agent used for the measuring method using a labeled substance are radioisotopes, enzymes, fluorescent substances, luminescent substances and the like. Examples of radioisotopes used are [¹²⁵I], [¹³¹I], [³H], [¹⁴C], [³²P], [³³P], [³⁵S] and the like. As the aforementioned enzymes, stable enzymes of high specific activity are preferred; for example, β-galactosidase, β-glucosidase, alkaline phosphatase, peroxidase, malate dehydrogenase and the like are used. Examples of fluorescent substances used include fluorescamine, fluorescein isothiocyanate, cyanin fluorescent dyes and the like. Examples of luminescent substances used are luminol, luminol derivatives, luciferin, lucigenin and the like. Furthermore, a biotin-(strepto)avidin system may also be used for binding an antibody or antigen and a labeling agent.

The method for quantifying CPSF5 and CPSF6 proteins using an antibody for detection of the present invention are not to be limited particularly; any method of measurement can be used, as far as the amount of antibody, antigen or antibody-antigen complex corresponding to the amount of antigen in a sample liquid can be detected by a chemical or physical means, and can be calculated from a standard curve generated using standard solutions containing known amounts of the antigen. For example, nephelometry, the competitive method, immunometric method, and sandwich method are advantageously used. For example, the sandwich method described below is preferable in terms of sensitivity and specificity.

In the immobilization of antigens or antibodies, physical adsorption may be used. Alternatively, chemical binding that is conventionally used for immobilization/stabilization of proteins, enzymes, etc. may be used as well. Examples of the carrier include insoluble polysaccharides such as agarose, dextran, cellulose, etc.; synthetic resins such as polystyrene, polyacrylamide, silicone, etc.; or glass; and the like.

In the sandwich method, an antibody for detection of the present invention insolubilized is reacted with a sample liquid (primary reaction), then reacted with another antibody for detection of the present invention labeled (secondary reaction), after which the amount or activity of the labeling agent on the insolubilizing carrier is measured, whereby CPSF5 or CPSF6 protein in the test liquid can be quantified. The primary reaction and the secondary reaction may be performed in the reverse order, or performed simultaneously, or performed with a time lag. The labeling agent and the method of insolubilization can be the same as those described above. In the immunoassay by the sandwich method, the antibody used as the immobilized antibody or the labeled antibody does not always need to be from one kind, but a mixture of two or more kinds of antibodies may be used for the purpose of increasing the measurement sensitivity and the like.

An antibody for detection of the present invention can be used in measurement systems other than the sandwich method, for example, the competitive method, immunometric method, nephelometry and the like.
In the competitive method, two proteins of CPSF5 or CPSF6 protein and labeled CPSF5 or CPSF6 protein are competitively reacted with an antibody in a sample liquid, after which the unreacted labeled antigen (F) and the labeled antigen bound to the antibody (B) are separated (B/F separation), and the amount labeled in B or F is measured, whereby the CPSF5 or CPSF6 protein in the sample liquid is quantified. This reaction method includes a liquid phase method using a soluble antibody as the antibody, polyethylene glycol and a secondary antibody to the aforementioned antibody (primary antibody) and the like to achieve B/F separation, and an immobilization method using an immobilized antibody as the primary antibody (direct method), or using a soluble antibody as the primary antibody and an immobilized antibody as the secondary antibody (indirect method).
In the immunometric method, the CPSF5 or CPSF6 protein in a sample liquid and the CPSF5 or CPSF6 protein immobilized are competitively reacted with a given amount of a labeled antibody, after which the solid phase and the liquid phase are separated, or the CPSF5 or CPSF6 protein in a sample liquid and an excess amount of a labeled antibody are reacted, and then the CPSF5 or CPSF6 protein immobilized is added to bind the unreacted portion of the labeled antibody to the solid phase, after which the solid phase and the liquid phase are separated. Next, the amount labeled in either phase is measured to quantify the amount of antigen in the sample liquid.
In nephelometry, the amount of insoluble precipitate resulting from an antigen-antibody reaction in a gel or in a solution is measured. Even when the amount of the CPSF5 or CPSF6 protein in the sample liquid is so small that only a small amount of precipitate is obtained, laser nephelometry, which utilizes laser scattering, and the like are suitably used.

In applying these individual immunological measurement methods to the method of quantification of the present invention, it is unnecessary to set special conditions, procedures and the like. Making ordinary technical considerations for those skilled in the art to the ordinary conditions and procedures in each method, a measurement system for CPSF5 and CPSF6 protein can be constructed. For details of these general technical means, compendia, books and the like can be referred to.
For example, Hiroshi Irie, ed., "Radioimmunoassay" (Kodansha Ltd., published in 1974), Hiroshi Irie, ed., "Sequel to the Radioimmunoassay" (Kodansha Ltd., published in 1979), Eiji Ishikawa et al., ed., "Enzyme Immonoassay" (Igakushoin, published in 1978), Eiji Ishikawa et al., ed., "Enzyme Immonoassay" (2nd ed.) (Igakushoin, published in 1982), Eiji Ishikawa et al., ed., "Enzyme Immonoassay" (3rd ed.) (Igakushoin, published in 1987), Methods in ENZYMOLOGY, Vol. 70 (Immunochemical Techniques (Part A)), ibidem, Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibidem, Vol. 92 (Immunochemical Techniques (Part E: Monoclonal Antibodies and General Immunoassay Methods)), ibidem, Vol. 121 (Immunochemical Techniques (Part I: Hybridoma Technology and Monoclonal Antibodies)) (all published by Academic Press Publishing) and the like.
By using an antibody for detection of the present invention as described above, the amounts of the CPSF5 and CPSF6 proteins in cells can be quantified with high sensitivity.

For example, in the above-described screening methods (a) and (b), if the expression level (amount of mRNA or amount of protein) of CPSF5 and/or CPSF6 in the presence of a test compound, compared with the level in the absence of the test compound, is inhibited by about 20% or more, preferably about 30% or more, more preferably about 50% or more, the test compound or a salt thereof can be selected as a candidate for a substance that inhibits the expression of CPSF5 and/or CPSF6 protein, and hence as a substance possessing insulin resistance ameliorating action.

Obtained using a screening method of the present invention, a substance that inhibits the expression and/or activity of CPSF5 and a substance that inhibits the expression and/or activity of CPSF6 (may be a free form or in the form of a salt) can be used as, for example, insulin sensitizers, gluconeogenesis inhibitors and the like, as, for example, prophylactic/therapeutic agents for diseases associated with sugar metabolism abnormality [e.g., diabetes (preferably type II diabetes), diabetic complications (e.g., neuropathy, nephropathy, retinitis and the like), impaired glucose intolerance, obesity, metabolic syndrome and the like], diseases associated with lipid metabolism abnormality [e.g., arteriosclerosis, hypertension, hyperlipemia (particularly hypertriglyceridemia and the like), fatty liver, non-alcoholic steatohepatitis (NASH), sudden cardiac death, nonfatal myocardial infarction, resting angina pectoris/angina of effort, cardiovascular diseases (e.g., angina pectoris instabilization and the like), cerebrovascular disorders (e.g., cerebral thrombosis, cerebral embolism, cerebral hemorrhage, subarachnoid hemorrhage, transient cerebral ischemic attack and the like) and the like].
When a substance obtained using a screening method of the present invention is used as a prophylactic/therapeutic agent as described above, the substance can be prepared in the same manner as with the above-described low-molecular compound that inhibits the expression and/or activity of CPSF5 (or CPSF6), and can be administered orally or parenterally, with similar routes of administration and doses, to humans or mammals (for example, mice, rats, rabbits, sheep, pigs, bovines, horses, cats, dogs, monkeys, chimpanzees and the like).

In the specification, where bases, amino acids, etc. are denoted by their codes, they are based on conventional codes in accordance with the IUPAC-IUB Commission on Biochemical Nomenclature or by the common codes in the art, examples of which are shown below. For amino acids that may have the optical isomer, L form is presented unless otherwise indicated.
- DNA: : deoxyribonucleic acid
- DNA :: complementary deoxyribonucleic acid
- A: : adenine
- T: : thymine
- G: : guanine
- C: : cytosine
- RNA: : ribonucleic acid
- RNA :: messenger ribonucleic acid
- dATP :: deoxyadenosine triphosphate
- dTTP :: deoxythymidine triphosphate
- dGTP :: deoxyguanosine triphosphate
- dCTP :: deoxycytidine triphosphate
- ATP: : adenosine triphosphate
- EDTA :: ethylenediaminetetraacetic acid
- SDS: : sodium dodecyl sulfate
- Gly: : glycine
- Ala: : alanine
- Val: : valine
- Leu: : leucine
- Ile: : isoleucine
- Ser: : serine
- Thr: : threonine
- Cys: : cysteine
- Met: : methionine
- Glu: : glutamic acid
- Asp: : aspartic acid
- Lys: : lysine
- Arg: : arginine
- His: : histidine
- Phe: : phenylalanine
- Tyr: : tyrosine
- Trp: : tryptophan
- Pro: : proline
- Asn: : asparagine
- Gln: : glutamine
- pGlu :: pyroglutamic acid
- Sec: : selenocysteine

The sequence identification numbers in the sequence listing herein indicate the following sequences.
[SEQ ID NO:1]
   Shows the base sequence of a cDNA that encodes CPSF5.
[SEQ ID NO:2]
   Shows the amino acid sequence of CPSF5.
[SEQ ID NO:3]
   Shows the base sequence of a cDNA that encodes CPSF6.
[SEQ ID NO:4]
   Shows the amino acid sequence of CPSF6.
[SEQ ID NO:5]
   Shows the base sequence of a target sequence for an siRNA against CPSF5, CPSF5-1.
[SEQ ID NO:6]
   Shows the base sequence of a sense chain of an siRNA against CPSF5, CPSF5-1.
[SEQ ID NO:7]
   Shows the base sequence of an antisense chain of an siRNA against CPSF5, CPSF5-1.
[SEQ ID NO:8]
   Shows the base sequence of a target sequence for an siRNA against CPSF6, CPSF6-1.
[SEQ ID NO:9]
   Shows the base sequence of a sense chain of an siRNA against CPSF6, CPSF6-1.
[SEQ ID NO:10]
   Shows the base sequence of an antisense chain of an siRNA against CPSF6, CPSF6-1.
[SEQ ID NO:11]
   Shows the base sequence of a target sequence for an siRNA against CPSF5, CPSF5-2.
[SEQ ID NO:12]
   Shows the base sequence of a sense chain of an siRNA against CPSF5, CPSF5-2.
[SEQ ID NO:13]
   Shows the base sequence of an antisense chain of an siRNA against CPSF5, CPSF5-2.
[SEQ ID NO:14]
   Shows the base sequence of a target sequence for an siRNA against CPSF5, CPSF5-3.
[SEQ ID NO:15]
   Shows the base sequence of a sense chain of an siRNA against CPSF5, CPSF5-3.
[SEQ ID NO:16]
   Shows the base sequence of an antisense chain of an siRNA against CPSF5, CPSF5-3.
[SEQ ID NO:17]
   Shows the base sequence of a target sequence for an siRNA against CPSF5, CPSF5-4.
[SEQ ID NO:18]
   Shows the base sequence of a sense chain of an siRNA against CPSF5, CPSF5-4.
[SEQ ID NO:19]
   Shows the base sequence of an antisense chain of an siRNA against CPSF5, CPSF5-4.
[SEQ ID NO:20]
   Shows the base sequence of a target sequence for an siRNA against CPSF6, CPSF6-2.
[SEQ ID NO:21]
   Shows the base sequence of a sense chain of an siRNA against CPSF6, CPSF6-2.
[SEQ ID NO:22]
   Shows the base sequence of an antisense chain of an siRNA against CPSF6, CPSF6-2.
[SEQ ID NO:23]
   Shows the base sequence of a target sequence for an siRNA against CPSF6, CPSF6-3.
[SEQ ID NO:24]
   Shows the base sequence of a sense chain of an siRNA against CPSF6, CPSF6-3.
[SEQ ID NO:25]
   Shows the base sequence of an antisense chain of an siRNA against CPSF6, CPSF6-3.
[SEQ ID NO:26]
   Shows the base sequence of a target sequence for an siRNA against CPSF6, CPSF6-4.
[SEQ ID NO:27]
   Shows the base sequence of a sense chain of an siRNA against CPSF6, CPSF6-4.
[SEQ ID NO:28]
   Shows the base sequence of an antisense chain of an siRNA against CPSF6, CPSF6-4.
[SEQ ID NO:29]
   Shows the base sequence of a sense primer for CPSF5.
[SEQ ID NO:30]
   Shows the base sequence of an antisense primer for CPSF5.
[SEQ ID NO:31]
   Shows the base sequence of a probe for CPSF5.
[SEQ ID NO:32]
   Shows the base sequence of a sense primer for CPSF6.
[SEQ ID NO:33]
   Shows the base sequence of an antisense primer for CPSF6.
[SEQ ID NO:34]
   Shows the base sequence of a probe for CPSF6.
[SEQ ID NO:35]
   Shows the base sequence of a sense primer for β-actin.
[SEQ ID NO:36]
   Shows the base sequence of an antisense primer for β-actin.
[SEQ ID NO:37]
   Shows the base sequence of a probe for β-actin.

### [Examples]

The present invention is hereinafter described more specifically by means of the following working examples and reference examples, to which, however, the invention is never limited.

### Example 1

### (1) Selection of the clones, H4IIE-C3-No.75 and 76 strains from a rat hepatoma cell line H4IIE-C3

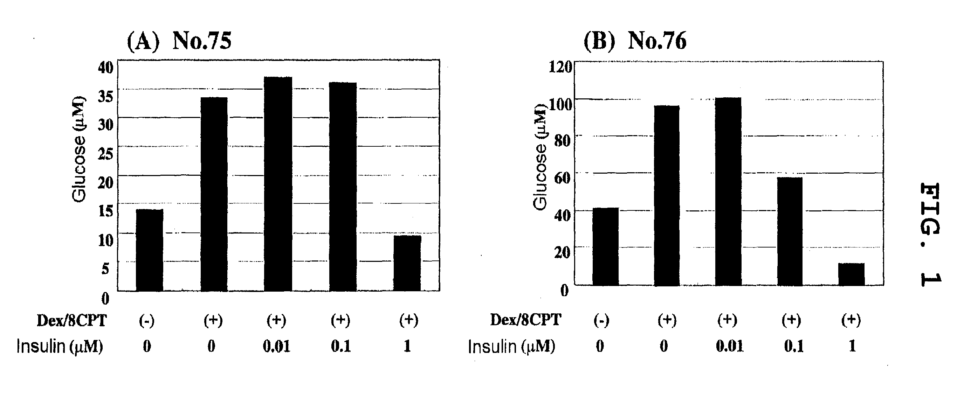

By limiting dilution from a rat hepatoma cell line, H4IIE-C3 strain (Dainippon Pharmaceutical), H4IIE-C3-No.75 and H4IIE-C3-No.76 cells were isolated. For these clones, inhibitory effects of glucose production by insulin were evaluated as follows.
H4IIE-C3-No.75 and H4IIE-C3-No.76 cells were separately plated on a type 1 collagen-coated 24-well plate (Nippon Becton Dickinson) at a concentration of 5x10⁵ cells/well. After the H4IIE-C3-No.75 or H4IIE-C3-No.76 cells were cultured in a growth medium (Dulbecco Modified Essential Medium F12 (DMEM/F12) + 10% Fetal Bovine Serum (FBS) for 48 hours, the medium was replaced with a serum-free medium (DMEM). After 24 hours cultivation, the medium was further replaced with an insulin-supplemented glucose production buffer (GPB: a phenol red-free and glucose-free DMEM containing 20mM sodium lactate, 1mM sodium pyruvate, and 15mM HEPES (pH 7.5)), and the cells were incubated for 1 hour. Furthermore, 500nM dexamethasone (Dex) and 100 µM 8-(4-CHLOROPHENYLTHIO)-ADENOSINE 3':5'-CYCLIC MONOPHOSPHATE SODIUM SALT (8CPT) (Calbiochem) were added and the next day the culture supernatant was recovered. The glucose concentration in the culture supernatant obtained was determined using the Amplex red Glucose oxidase assay kit (Molecular Probes). The ED₅₀ values of the insulin resistant strain H4IIE-C3-No.75 and the insulin sensitive strain H4IIE-C3-No.76 for the inhibitory effects of the Dex/8CPT-stimulated glucose production by insulin, were 330nM and 60nM, respectively (FIG. 1).

### (2) Preparation of an RNA-related factors-focused siRNA library

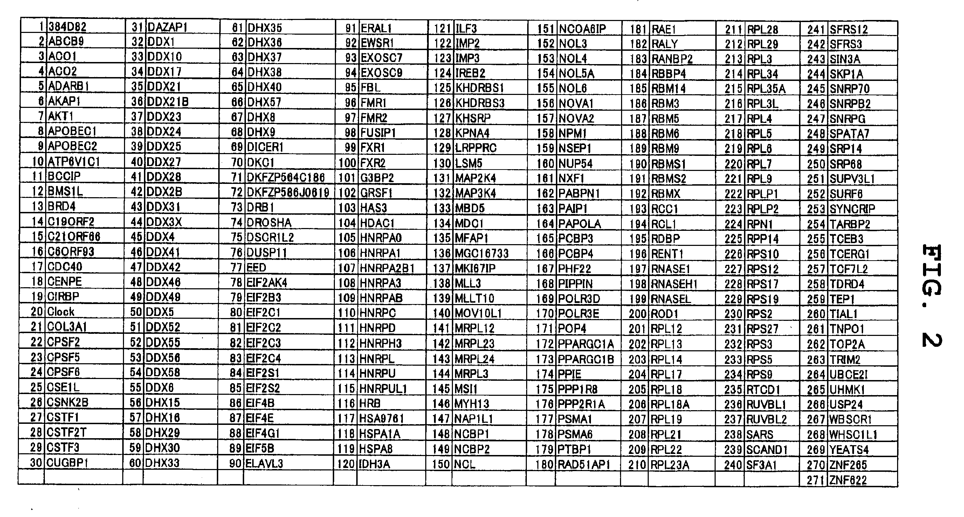

Mammalian homologues of 90 genes reported by screening for the whole genome in C. elegans using siRNAs to play an important role in RNA interference effect (Science Vol.308, p.1164, 2005), known RISC-related factors, RNA helicases and RNA-binding proteins and the like were selected as candidates for RNA-related factors. Selected RNA-related factors are shown in FIG. 2. The rat siRNAs of 271 genes shown in FIG. 2 were purchased from Ambion and Qiagen, and used as the library of RNA-related factors.

### (3) Construction of a screening system using H4IIE-C3-No.75 strain

Each of the 271 siRNAs in the above-described library of RNA-related factors was introduced into the insulin resistant strain H4IIE-C3-No.75 by electroporation method. Electroporation was performed using Nucleofector II (Amaxa) in combination with the reagent Nucleofector T and the program T-27 (Amaxa). The cell density was 4x10⁶ cells/cuvette. 20µM siRNA was used at 16 µl/cuvette. After the electroporation, the cells were cultured for 48 hours in the proliferation medium, and then incubated in the serum-free medium for 24 hours. Furthermore, the cells were twice washed with Phosphate Buffered Saline (PBS), which was then replaced with GPB, after which insulin stimulation and Dex/8CPT stimulation were performed at a final concentration of 100nM for 24 hours, after which the culture supernatant was recovered, and glucose concentrations were measured using the Amplex red Glucose oxidase assay kit (Molecular Probes). In each of three cases, one without any stimulation, one with Dex/8CPT stimulation and without insulin stimulation, and one with Dex/8CPT stimulation and with insulin stimulation, glucose concentrations were measured. siRNAs that inhibited glucose production more potently with insulin stimulation than without insulin stimulation were screened for, and an siRNA against CPSF5 (CPSF5-1) and an siRNA against CPSF6 (CPSF6-1) were selected.
a) CPSF5-1
   Target sequence: 5'-CCGTATATTCCTGCACATATA-3' (SEQ ID NO:5)
   Sense chain: 5'-r(GUAUAUUCCUGCACAUAUA)dTdT-3' (SEQ ID NO:6)
   Antisense chain: 5'-r(UAUAUGUGCAGGAAUAUAC)dGdG-3' (SEQ ID NO:7)
b) CPSF6-1
   Target sequence: 5'-TAGATGTAGTGTTGTAATAAA-3' (SEQ ID NO:8)
   Sense chain: 5'-r(GAUGUAGUGUUGUAAUAAA)dTdT-3' (SEQ ID NO:9)
   Antisense chain: 5'-r(UUUAUUACAACACUACAUC)dTdA-3' (SEQ ID NO:10)

### (4) Evaluation of glucose production by siRNA against CPSF5 and siRNA against CPSF6

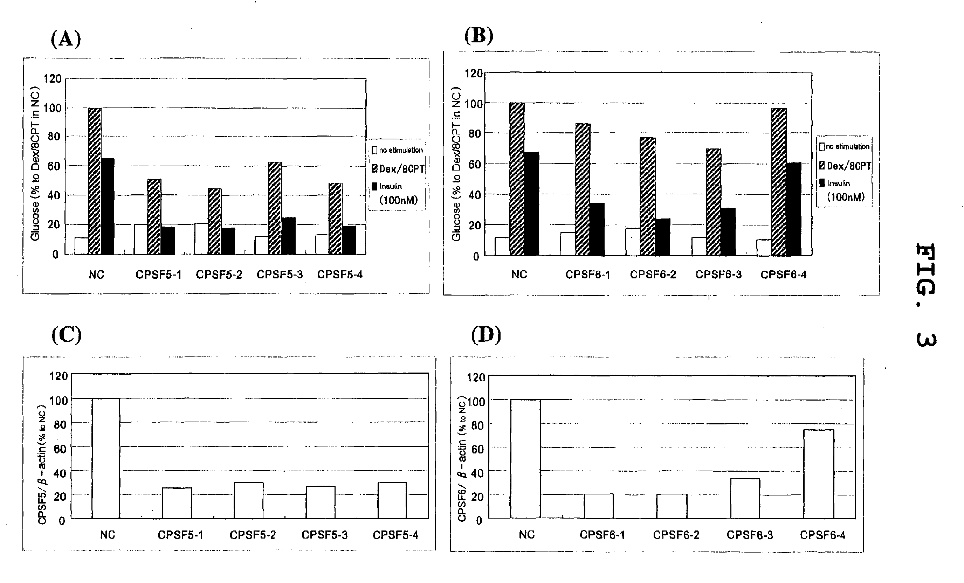

The inhibitory effects of CPSF5 and CPSF6 on sugar production with insulin stimulation were examined, using the plurality of siRNAs shown below, respectively. As a result, as shown in the upper panels in FIG. 3, it was confirmed that a plurality of siRNAs, specifically CPSF5-1, 2, 3, and 4 and CPSF6-1, 2, 3, and 4, were effective in inhibiting glucose production with insulin stimulation.
i) siRNAs against CPSF5
   a) CPSF5-1
      Target sequence: 5'-CCGTATATTCCTGCACATATA-3' (SEQ ID NO:5)
      Sense chain: 5'-r(GUAUAUUCCUGCACAUAUA)dTdT-3' (SEQ ID NO:6)
      Antisense chain: 5'-r(UAUAUGUGCAGGAAUAUAC)dGdG-3' (SEQ ID NO:7)
   b) CPSF5-2
      Target sequence: 5'-CTGGTTCAGCTTCAAGAGAAA-3' (SEQ ID NO:11)
      Sense chain: 5'-r(GGUUCAGCUUCAAGAGAAA)dTdT-3' (SEQ ID NO:12)
      Antisense chain: 5'-r(UUUCUCUUGAAGGUGAACC)dAdG-3' (SEQ ID NO:13)
   c) CPSF5-3
      Target sequence: 5'-CGGGAGGAATTTGATAAGATT-3' (SEQ ID NO:14)
      Sense chain: 5'-r(GGAGGAAUUUGAUAAGAUU)dTdT-3' (SEQ ID NO:15)
      Antisense chain: 5'-r(AAUCUUAUCAAAUUCCUCC)dCdG-3' (SEQ ID NO:16)
   d) CPSF5-4
      Target sequence: 5'-CCAGGAGAAGATGAAGTTGAA-3' (SEQ ID NO:17)
      Sense chain: 5'-r(AGGAGAAGAUGAAGUUGAA)dTdT-3' (SEQ ID NO:18)
      Antisense chain: 5'-r(UUCAACUUCAUCUUCUCCU)dGdG-3' (SEQ ID NO:19)
ii) siRNAs against CPSF6
   a) CPSF6-1
      Target sequence: 5'-TAGATGTAGTGTTGTAATAAA-3' (SEQ ID NO:8)
      Sense chain: 5'-r(GAUGUAGUGUUGUAAUAAA)dTdT-3' (SEQ ID NO:9)
      Antisense chain: 5'-r(UUUAUUACAACACUACAUC)dTdA-3' (SEQ ID NO:10)
   b) CPSF6-2
      Target sequence: 5'-CACGGTCAGAATCCTGTTGTA-3' (SEQ ID NO:20)
      Sense chain: 5'-r(CGGUCAGAAUCCUGUUGUA)dTdT-3' (SEQ ID NO:21)
      Antisense chain: 5'-r(UACAACAGGAUUCUGACCG)dTdG-3' (SEQ ID NO:22)
   c) CPSF6-3
      Target sequence: 5'-ATCGGGCAAATGGACAATCAA-3' (SEQ ID NO:23)
      Sense chain: 5'-r(CGGGCAAAUGGACAAUCAA)dTdT-3' (SEQ ID NO:24)
      Antisense chain: 5'-r(UUGAUUGUCCAUUUGCCCG)dAdT-3' (SEQ ID NO:25)
   d) CPSF6-4
      Target sequence: 5'-AACGTGCAATATGCAAATAAT-3' (SEQ ID NO:26)
      Sense chain: 5'-r(CGUGCAAUAUGCAAAUAAU)dTdT-3' (SEQ ID NO:27)
      Antisense chain: 5'-r(AUUAUUUGCAUAUUGCACG)dTdT-3' (SEQ ID NO:28)

### (5) Taqman analysis of knock-down of CPSF5 and CPSF6 mRNAs

The amounts of the mRNAs of CPSF5, CPSF6 and β-action in RNAs extracted from the H4IIE-C3-No.75 strain incorporating the above-described siRNAs introduced according to the method (3) were measured by the Taqman PCR method. The Taqman PCR method was performed using the primers and probes shown below, according to the standard method specified by ABI. The results are shown in the lower panels in FIG. 3.
i) Against CPSF5
   Sense primer: 5'-ACCGTTGTTTGAACTGTACGACA-3' (SEQ ID NO:29)
   Antisense primer: 5'-CCTGCTCAGCAGCTGAGGA-3' (SEQ ID NO:30)
   Probe: 5'-FAM-TCCGGGATACGGACCCATCATTTCTAGT-TAMURA-3' (SEQ ID NO:31)
ii) Against CPSF6
   Sense primer: 5'-AGCTTGTGATTTTGCTGAATGG-3' (SEQ ID NO:32)
   Antisense primer: 5'-TTTTTTGACCCCTAACACATTGAA-3' (SEQ ID NO:33)
   Probe: 5'-FAM-ATGTAAACGTGTAAAAACTGAAATCTGACAGAGCAATC-TAMURA-3' (SEQ ID NO:34)
iii) Against β-acting
   Sense primer: 5'-TCCTGGCCTCACTGTCCAC-3' (SEQ ID NO:35)
   Antisense primer: 5'-GGGCCGGACTCATCGTACT-3' (SEQ ID NO:36)
   Probe: 5'-FAM-TTCCAGCAGATGTGGATCAGCAAGCA-TAMURA-3' (SEQ ID NO:37)

Introduction of CPSF5 and CPSF6 siRNAs enhanced the inhibition of Dex/8CPT-stimulated and insulin-stimulated glucose production without largely influencing Dex/8CPT-stimulated glucose production, i.e.,improved insulin resistance.
This action for a recovery from insulin resistance correlated with the knockdown efficiency of the target gene in the experiments using respective siRNAs. For example, the siRNA CPSF6-4 was less effective in sugar production suppression, and this is attributable to the lower knockdown effect of the siRNA on the CPSF6 gene. Because a plurality of siRNAs against the same gene exhibited the same action, it was shown that this action was not due to the off-target effect of the siRNAs, i.e., this effect was due to the knockdown effects for the CPSF5 and CPSF6 genes (FIG. 3). Hence, it was shown that by inhibiting the expression or function of CPSF5 and CPSF6, amelioration of insulin resistance and prevention/treatment of diabetes are possible.

### [Industrial Applicability]

A substance that inhibits the expression or activity of CPSF5 and a substance that inhibits the expression or activity of CPSF6 suppress insulin-stimulated gluconeogenesis on one hand, but do not influence Dex/8CPT-stimulated sugar production. This shows that the substances are capable of ameliorating insulin resistance without causing toxic signs such as lactate acidosis. Therefore, the substances are useful as safe and effective anti-diabetic drugs and the like.

Many cases have been known wherein a plurality of sites for mRNA precursor 3' end processing are present in a single gene (Genome Biol. 6,R100 (2005)); it has been reported that if the CPSF5 gene is knocked down using an siRNA against CPSF5, a plurality of cleavage sites in the 3'-UTR of the mRNA of a gene shift toward the 5' side, resulting in the formation of an mRNA with a shorter 3'-UTR (Nucleic Acids Res. 34,6264 (2006)). In the case of a gene wherein there is only one cleavage site in the 3'-UTR thereof, the length of the 3'-UTR remains unchanged even when CPSF5 is knocked down, so that CPSF5 can be said to contribute to the determination of the length of the 3'-UTR of the mRNA precursor for a particular gene.
Meanwhile, a microRNA (non-coding RNA consisting of 21-23 base pairs) is known to suppress the translation of a gene by recognizing a particular sequence present in the 3'-UTR of the mRNA of the gene. Regarding a gene under the control of a microRNA, if the length of the 3'-UTR of the mRNA shortens to result in the loss of the recognition sequence thereof, the gene no longer undergoes translational suppression by the microRNA, so that the expression level of the protein possibly increases.
Judging from these facts, a substance that inhibits the expression or activity of CPSF5 is possibly enhancing insulin sensitivity and exhibiting antidiabetic action by shortening the length of the 3'-UTR of a certain gene for insulin sensitivity enhancing action to cancel the translational suppression of a particular gene by a certain microRNA in the diabetic condition, resulting in an increase in a particular protein. Regarding CPSF6, the same possibility is suggested because it is also a constituent of the same CFIₘ complex.
As stated above, CPSF5 and CPSF6 are generally responsible for the processing at the mRNA precursor 3' end, and the influence of inhibition of the expression or activity thereof is limited to the expression of a particular gene; therefore, a substance that inhibits the expression or activity of CPSF5 or CPSF6 is thought to be of low toxicity and to be capable of selectively acting on the enhancement of the expression of a particular gene that exhibits insulin sensitivity enhancing action.

While the present invention has been described with emphasis on preferred embodiments, it is obvious to those skilled in the art that the preferred embodiments can be modified. The present invention intends that the present invention can be embodied by methods other than those described in detail in the present specification. Accordingly, the present invention encompasses all modifications encompassed in the gist and scope of the appended "CLAIMS."
This application is based on patent application No. 2007-039947 filed in Japan (filing date: February 20, 2007), and the contents disclosed therein are hereby entirely incorporated by reference. In addition, the contents disclosed in any publication cited herein, including patents and patent applications, are hereby incorporated in their entireties by reference, to the extent that they have been disclosed herein.

## Claims

1. An insulin sensitizer comprising a substance inhibiting expression or activity of a protein comprising an amino acid sequence which is the same or substantially the same as the amino acid sequence shown by SEQ ID NO: 2, and/or a substance inhibiting expression or activity of a protein comprising an amino acid sequence which is the same or substantially the same as the amino acid sequence shown by SEQ ID NO: 4.

2. The sensitizer of claim 1, wherein the substance inhibiting expression of a protein comprising an amino acid sequence which is the same or substantially the same as the amino acid sequence shown by SEQ ID NO: 2, and/or the substance inhibiting expression of a protein comprising an amino acid sequence which is the same or substantially the same as the amino acid sequence shown by SEQ ID NO: 4 are/is any of the following (a) to (c):
(a) an antisense nucleic acid to a nucleic acid encoding each protein
(b) siRNA to RNA encoding each protein
(c) a nucleic acid capable of producing siRNA to RNA encoding each protein.

3. The sensitizer of claim 1 having a gluconeogenesis inhibitory action.

4. The sensitizer of claim 1, which is an agent for the prophylaxis or treatment of a disease involving a glucose metabolism disorder.

5. A method of improving insulin resistance in an animal, comprising administering, to the animal, (an) effective amount(s) of a substance inhibiting expression or activity of a protein comprising an amino acid sequence which is the same or substantially the same as the amino acid sequence shown by SEQ ID NO: 2, and/or a substance inhibiting expression or activity of a protein comprising an amino acid sequence which is the same or substantially the same as the amino acid sequence shown by SEQ ID NO: 4.

6. Use of a substance inhibiting expression of a protein comprising an amino acid sequence which is the same or substantially the same as the amino acid sequence shown by SEQ ID NO: 2, and/or a substance inhibiting expression of a protein comprising an amino acid sequence which is the same or substantially the same as the amino acid sequence shown by SEQ ID NO: 4, for the production of an insulin sensitizer.

7. A method of screening for an insulin sensitizing substance, comprising contacting cells producing the following (a) and/or (b) :
(a) a protein comprising an amino acid sequence which is the same or substantially the same as the amino acid sequence shown by SEQ ID NO: 2 or a partial peptide thereof
(b) a protein comprising an amino acid sequence which is the same or substantially the same as the amino acid sequence shown by SEQ ID NO: 4 or a partial peptide thereof
with a test compound, and measuring an expression level or activity of the protein of said (a) or a partial peptide thereof and/or the protein of said (b) or a partial peptide thereof.

8. The method of claim 7, wherein the insulin sensitizing substance has a gluconeogenesis inhibitory action.

9. The method of claim 7, wherein the insulin sensitizing substance can prevent or treat a disease involving a glucose metabolism disorder.


SEQUENCE LISTING

<110>  Takeda Pharmaceutical Company Limited

<120>  AMELIORATING AGENT FOR INSULIN RESISTANCE

<130>  091199

<150>  JP 2007-039947
<151>  2007-02-20

<160>  37

<170>  PatentIn version 3.3

<210>  1
<211>  4407
<212>  DNA
<213>  Homo sapiens


<220>
<221>  CDS
<222>  (148)..(828)

<400>  1
ttccggcgtg cctacgcctc ctcttgcgct gtcctgttaa tggcgggcag tagccgctga      60

ggggattgca gataaccgct tcccgcacgg ggaaagtcta ccctgcctgc cactttctgc     120

tcgccgtcag cgccggagct cgccagc atg tct gtg gta ccg ccc aat cgc tcg     174
                              Met Ser Val Val Pro Pro Asn Arg Ser
                              1               5

cag acc ggc tgg ccc cgg ggg gtc act cag ttc ggc aac aag tac atc       222
Gln Thr Gly Trp Pro Arg Gly Val Thr Gln Phe Gly Asn Lys Tyr Ile
10                  15                  20                  25

cag cag acg aag ccc ctc acc ctg gag cgc acc atc aac ctg tac cct       270
Gln Gln Thr Lys Pro Leu Thr Leu Glu Arg Thr Ile Asn Leu Tyr Pro
                30                  35                  40

ctt acc aat tat act ttt ggt aca aaa gag ccc ctc tac gag aag gac       318
Leu Thr Asn Tyr Thr Phe Gly Thr Lys Glu Pro Leu Tyr Glu Lys Asp
            45                  50                  55

agc tct gtt gca gcc aga ttt cag cgc atg agg gaa gaa ttt gat aaa       366
Ser Ser Val Ala Ala Arg Phe Gln Arg Met Arg Glu Glu Phe Asp Lys
        60                  65                  70

att gga atg agg agg act gta gaa ggg gtt ctg att gta cat gag cac       414
Ile Gly Met Arg Arg Thr Val Glu Gly Val Leu Ile Val His Glu His
    75                  80                  85

cgg cta ccc cat gtg tta ctg ctg cag ctg gga aca act ttc ttc aaa       462
Arg Leu Pro His Val Leu Leu Leu Gln Leu Gly Thr Thr Phe Phe Lys
90                  95                  100                 105

cta cct ggt ggt gaa ctt aac cca gga gaa gat gaa gtt gaa gga cta       510
Leu Pro Gly Gly Glu Leu Asn Pro Gly Glu Asp Glu Val Glu Gly Leu
                110                 115                 120

aaa cgc tta atg aca gag ata ctg ggt cgt cag gat gga gtt ttg caa     558
Lys Arg Leu Met Thr Glu Ile Leu Gly Arg Gln Asp Gly Val Leu Gln
            125                 130                 135

gac tgg gtc att gac gat tgc att ggt aac tgg tgg aga cca aat ttt     606
Asp Trp Val Ile Asp Asp Cys Ile Gly Asn Trp Trp Arg Pro Asn Phe
        140                 145                 150

gaa cct cct cag tat cca tat att cct gca cat att aca aag cct aag     654
Glu Pro Pro Gln Tyr Pro Tyr Ile Pro Ala His Ile Thr Lys Pro Lys
    155                 160                 165

gaa cat aag aag ttg ttt ctg gtt cag ctt caa gaa aaa gcc ttg ttt     702
Glu His Lys Lys Leu Phe Leu Val Gln Leu Gln Glu Lys Ala Leu Phe
170                 175                 180                 185

gca gtc cct aaa aat tac aag ctg gta gct gca cca ttg ttt gaa ttg     750
Ala Val Pro Lys Asn Tyr Lys Leu Val Ala Ala Pro Leu Phe Glu Leu
                190                 195                 200

tat gac aat gca cca gga tat gga ccc atc att tct agt ctc cct cag     798
Tyr Asp Asn Ala Pro Gly Tyr Gly Pro Ile Ile Ser Ser Leu Pro Gln
            205                 210                 215

ctg ttg agc agg ttc aat ttt att tac aac tgaattcctg cgcagtggag       848
Leu Leu Ser Arg Phe Asn Phe Ile Tyr Asn
        220                 225

aagtaaaaga agccgcttgt ctctgtgagc acagctatat acagtgtaga ataaatgtgg    908

tagaaaagtt tttttggttt tatctctttt gcgatcccta aattgccacc tttctattgt    968

ttgaatagta aaattaatat gaagaactag atagtggtgt aaacaaatgt gataatgttt   1028

atttactttc ggttctgctc atactttttt gtacaacatt aaagaaaatg gacttttttt   1088

attttaattt ctcattaaac ttctaaaatt cttataggtg aggatcattt ttccccccac   1148

cttaggatgg tgaatgttgc aacacaatga caggtttaag tcagtcaagt ttattggacc   1208

cttgctttga taccattctt gggcacatac tccaagattg tattagattt ttgtgatgaa   1268

gagcttccat tacttctgaa aactatattt atctgagtga gtccaaggtg caactcctaa   1328

atgaattgtg ttgcagagaa ctcccagtat aattcactga ccagtacatt ttataaccat   1388

ccaggccttg gtttgcaagc aacagacctt aaacatacag gaaactatta aaattggctc   1448

gatcagtagt cataggaatt ggtataagaa gagactcatt tagagctcag agttttcttc   1508

acataatggg ggtattaatt atttgtgctg ttgcgaaatt atgtgtctta ttcttaaagc   1568

catggtaaaa atagggatct gtgaaggaaa tttctaaaat tggatgtatt aggttttgaa   1628

ctctgagatt gcacaaatat tcaattaact tgaagttgtg tacatagaga agaaaatttg   1688

gttttagcaa atgacagagc cttcaaaaat atttttggaa taatgtgaat caaccgaaaa   1748

ctgggggcaa ggcagaggac aggttttctc aggttaagag aaaaacgaaa ttttaaaaac   1808

tttaaaaaat actgataaat tcggatcaaa tttgggggaa taaaaaatat tagagcaaag 1868

gagtttgctg gttgtgtcat tatttaatga tcaaagtata gcatgtatgc cttattacag 1928

acttgttgac tataggctta atgtaaaaag gaatcttgcc agatgtagct acttaaggaa 1988

aaaagggttt ttaatagaaa tgaacttttg attagtatgg tgccagtcac agggctattt 2048

tcctgaatat tgggtgatgt caaaggtata tgatacttga ggaaatcagg ccaagtgtag 2108

ctgagcaatt aataaacact caagtttttt tagttggggt catgtcaaac cctcacatgg 2168

tggttaagta gtgactaact gtgcatgtcc ttcatggtag gtgagcacca gtctccatga 2228

tcaaaaatgc cccagtttcc tttgaaaaag ccactagttc tgtgaattgt ccttttccct 2288

cctgccccta acagtcctac ccctctagcc tacattagca tatttctcat gtaatgtatt 2348

ttgctggtaa gctcatccat tagctgcgtc cttcagctat tcctttagat tggaggaagt 2408

ggatatgaag atggattgat tcagctcacc ttccatgatt gcttttgagg gaaaagctac 2468

agtggccaca tttcagatgt tcacttttgc aatttgtgga gggtggagag aggtagactt 2528

ttgttgtgtg ttttataaag tactctcagt aaggagtgtt tgagttgaag agtcatctga 2588

ttcgaggcca ctcatgttct ttgtaactta aactttgacc aagaaattct tcacttctca 2648

cttcttcact tcttcccaat atacagtaag tacgtgagcc agtcatccat acactaaggc 2708

ctagttgaga aaaacctttg attcaggatg gctgggttac taaccttgaa atgtaagaga 2768

tctggttttg aatgtaaaag ttgcaacaca caaacggaag tcttaaaaac tttttgctct 2828

ggtcagttac aggtggatcc ccaataatct gtttttggtt ttctgatgga aataatagaa 2888

ttaggggaaa tcaaatctgg ttggtaggtg tctacagtat tagaagaggg tataagggca 2948

ctgtttaaca ctaagttcta atacttccag aaactgtgca ttccagatct acatactaaa 3008

tgctcttatc attttgaaat gggctcttga ttaatagacc catatttttt agtggcttct 3068

atgttgtata tttgtctaaa atgaaagctc ttttgcgttc taaaactaca atatatgtca 3128

tcttattttc cctgagtatc caagtatagt gcagattcta tgtaaaacta ctaaatgaca 3188

ctggaatatg tttagtagat tagggggaaa aactataaag gtttatacaa ttgtttgtag 3248

ttacatttag gatggactta tccctttgga gaagagtgaa gtttgttttt tcgccatgtg 3308

atgaagacca ctgtgatttt ttaaaaaagt agataatact taaaatggcg taataattct 3368

gcacttgaat ttgtactgtt aacagcacat ttggaagatt ttaaaacttt ttattgtctt 3428

ataaatagca ttcacttatt attttggata tttaagggtt ccattaagtt aacactgtat 3488

ttggacaaag tgtgaccaaa ttagccagtc tgttttcttc catgtttaat tagaagtgag 3548

aggtagaagt acttcaaatt caacaggcca gcaagcaatc ggcttaaaat tccctttctt 3608

aaatgttgtg ctcttatgtt ctcggctttt taatgacttt atttttacag tacttgttca 3668

gtcacttgag atgaaatgct tggggtagct tttccatcct caaacttaat gtttttacta   3728

gttcatagtg tttggaacag tatatgccaa tcactgagac tgcatcagag tttgcaattt   3788

tgtatgtttc attgccaaag aaggcttagt ggttgttgac tgtagtataa gtcagctttc   3848

tgtagcataa gatttgattt tcccatactt acttcacttg ttatacatca ctgattattt   3908

gggttaaact ggactcattt caagcagttt gcttttgttc aaatcgtgat gagaaaccta   3968

atactgtaat ttgatttgag ccataaaaca cattttaata ttagcttgta ttatagttat   4028

taagcttgtt tttgtggaaa aaaacttact aaaacctagg taactctaga ttaggccagt   4088

tcaggtgtat tttgtatctt agtaatggat catatcgtaa aaatagagat aagttgggaa   4148

gatatattga ttatgctgtt ctgttgaggg aaaggtcatg tatttagaaa tttaaacttt   4208

tggttattgt gttcacatca tagtattcaa gcatcattta tagtttggtt ttgagaactt   4268

ttctggtatt acgtttatgg caaatgtata aaagaaacaa gttttggtta tatttttata   4328

tttgtaaagt aagtttggtt aaagtgatca ctgttctttt tttattttat tgtcatttca   4388

ataaaaaata tttgaaaga                                                4407

<210>  2
<211>  227
<212>  PRT
<213>  Homo sapiens

<400>  2

Met Ser Val Val Pro Pro Asn Arg Ser Gln Thr Gly Trp Pro Arg Gly
1               5                   10                  15

Val Thr Gln Phe Gly Asn Lys Tyr Ile Gln Gln Thr Lys Pro Leu Thr
            20                  25                  30

Leu Glu Arg Thr Ile Asn Leu Tyr Pro Leu Thr Asn Tyr Thr Phe Gly
        35                  40                  45

Thr Lys Glu Pro Leu Tyr Glu Lys Asp Ser Ser Val Ala Ala Arg Phe
    50                  55                  60

Gln Arg Met Arg Glu Glu Phe Asp Lys Ile Gly Met Arg Arg Thr Val
65                  70                  75                  80

Glu Gly Val Leu Ile Val His Glu His Arg Leu Pro His Val Leu Leu
                85                  90                  95

Leu Gln Leu Gly Thr Thr Phe Phe Lys Leu Pro Gly Gly Glu Leu Asn
            100                 105                 110

Pro Gly Glu Asp Glu Val Glu Gly Leu Lys Arg Leu Met Thr Glu Ile
        115                 120                 125

Leu Gly Arg Gln Asp Gly Val Leu Gln Asp Trp Val Ile Asp Asp Cys
    130                 135                 140

Ile Gly Asn Trp Trp Arg Pro Asn Phe Glu Pro Pro Gln Tyr Pro Tyr
145                 150                 155                 160

Ile Pro Ala His Ile Thr Lys Pro Lys Glu His Lys Lys Leu Phe Leu
                165                 170                 175

Val Gln Leu Gln Glu Lys Ala Leu Phe Ala Val Pro Lys Asn Tyr Lys
            180                 185                 190

Leu Val Ala Ala Pro Leu Phe Glu Leu Tyr Asp Asn Ala Pro Gly Tyr
        195                 200                 205

Gly Pro Ile Ile Ser Ser Leu Pro Gln Leu Leu Ser Arg Phe Asn Phe
    210                 215                 220

Ile Tyr Asn
225

<210>  3
<211>  3426
<212>  DNA
<213>  Homo sapiens

<220>
<221>  CDS
<222>  (35)..(1687)

<400>  3
aattccgggc ggcggcggcc gaggctgaag gaag atg gcg gac ggc gtg gac cac      55
                                      Met Ala Asp Gly Val Asp His
                                      1               5

ata aac att tac gcg gat gtc ggc gaa gag ttc aac cag gaa gct gaa      103
Ile Asn Ile Tyr Ala Asp Val Gly Glu Glu Phe Asn Gln Glu Ala Glu
        10                  15                  20

tat ggt ggg cat gat cag ata gat ttg tat gac gat gtc ata tct cca      151
Tyr Gly Gly His Asp Gln Ile Asp Leu Tyr Asp Asp Val Ile Ser Pro
    25                  30                  35

tct gca aat aat gga gat gcc cca gaa gac cga gat tac atg gat act      199
Ser Ala Asn Asn Gly Asp Ala Pro Glu Asp Arg Asp Tyr Met Asp Thr
40                  45                  50                  55

ctc cca cca act gtt ggt gat gat gtg ggt aaa gga gca gca cca aat 247
Leu Pro Pro Thr Val Gly Asp Asp Val Gly Lys Gly Ala Ala Pro Asn
                60                  65                  70

gtt gtc tat aca tat act gga aag aga att gca tta tat att gga aat 295
Val Val Tyr Thr Tyr Thr Gly Lys Arg Ile Ala Leu Tyr Ile Gly Asn
            75                  80                  85

cta aca tgg tgg aca aca gat gaa gac tta act gaa gca gtt cat tct 343
Leu Thr Trp Trp Thr Thr Asp Glu Asp Leu Thr Glu Ala Val His Ser
        90                  95                  100

ttg gga gta aat gat att ttg gag ata aaa ttt ttt gaa aat cga gca 391
Leu Gly Val Asn Asp Ile Leu Glu Ile Lys Phe Phe Glu Asn Arg Ala
    105                 110                 115

aat ggc cag tca aag ggg ttt gcc ctt gtt ggt gtt gga tct gaa gca 439
Asn Gly Gln Ser Lys Gly Phe Ala Leu Val Gly Val Gly Ser Glu Ala
120                 125                 130                 135

tct tca aaa aag tta atg gat ctg tta cct aaa aga gaa ctt cat ggt 487
Ser Ser Lys Lys Leu Met Asp Leu Leu Pro Lys Arg Glu Leu His Gly
                140                 145                 150

cag aat cct gtt gta act cca tgc aat aaa cag ttc ctg agt caa ttt 535
Gln Asn Pro Val Val Thr Pro Cys Asn Lys Gln Phe Leu Ser Gln Phe
            155                 160                 165

gaa atg cag tcc agg aaa act aca caa tca gga caa atg tct ggg gaa 583
Glu Met Gln Ser Arg Lys Thr Thr Gln Ser Gly Gln Met Ser Gly Glu
        170                 175                 180

ggt aaa gct ggt cct cca gga ggc agt tcc cgt gca gca ttt cca caa 631
Gly Lys Ala Gly Pro Pro Gly Gly Ser Ser Arg Ala Ala Phe Pro Gln
    185                 190                 195

ggt ggt aga gga cgg ggc cgt ttt cca ggg gct gtt cct ggt ggg gac 679
Gly Gly Arg Gly Arg Gly Arg Phe Pro Gly Ala Val Pro Gly Gly Asp
200                 205                 210                 215

aga ttt cct ggg cca gca gga cca gga ggg cca ccc cca cct ttt cca 727
Arg Phe Pro Gly Pro Ala Gly Pro Gly Gly Pro Pro Pro Pro Phe Pro
                220                 225                 230

gct gga cag act cca cca cgt cca ccc tta ggt cct cca ggc cca cct 775
Ala Gly Gln Thr Pro Pro Arg Pro Pro Leu Gly Pro Pro Gly Pro Pro
            235                 240                 245

ggt cca cca ggt cct cca cct cct ggt cag gtt ctg cct cct cct cta 823
Gly Pro Pro Gly Pro Pro Pro Pro Gly Gln Val Leu Pro Pro Pro Leu
        250                 255                 260

gct ggg cct cct aat cga gga gat cgc cct cca cca cca gtt ctt ttt 871
Ala Gly Pro Pro Asn Arg Gly Asp Arg Pro Pro Pro Pro Val Leu Phe
    265                 270                 275

cct gga caa cct ttt ggg cag cct cca ttg ggt cca ctt cct cct ggc 919
Pro Gly Gln Pro Phe Gly Gln Pro Pro Leu Gly Pro Leu Pro Pro Gly
280                 285                 290                 295

cct cca cct cca gtt cca ggc tac ggc ccc cct cct ggc cca cca cct 967

Pro Pro Pro Pro Val Pro Gly Tyr Gly Pro Pro Pro Gly Pro Pro Pro
                300                 305                 310

cca caa cag gga cca cct cca cct cca ggc ccc ttt cca cct cgt cca      1015
Pro Gln Gln Gly Pro Pro Pro Pro Pro Gly Pro Phe Pro Pro Arg Pro
            315                 320                 325

ccc ggt cca ctt ggg cca ccc ctt aca cta gct cct cct ccg cat ctt      1063
Pro Gly Pro Leu Gly Pro Pro Leu Thr Leu Ala Pro Pro Pro His Leu
        330                 335                 340

cct gga cca cct cca ggt gcc cca ccg cca gct ccg cat gtg aac cca      1111
Pro Gly Pro Pro Pro Gly Ala Pro Pro Pro Ala Pro His Val Asn Pro
    345                 350                 355

gct ttc ttt cct cca cca act aac agt ggc atg cct aca tca gat agc      1159
Ala Phe Phe Pro Pro Pro Thr Asn Ser Gly Met Pro Thr Ser Asp Ser
360                 365                 370                 375

cga ggt cca cca cca aca gat cca tat ggg cga cct cca cca tat gat      1207
Arg Gly Pro Pro Pro Thr Asp Pro Tyr Gly Arg Pro Pro Pro Tyr Asp
                380                 385                 390

agg ggt gac tat ggc ccc cct gga agg gaa atg gat act gca aga acg      1255
Arg Gly Asp Tyr Gly Pro Pro Gly Arg Glu Met Asp Thr Ala Arg Thr
            395                 400                 405

cca ttg agt gaa gct gaa ttt gaa gaa atc atg aat aga aat agg gca      1303
Pro Leu Ser Glu Ala Glu Phe Glu Glu Ile Met Asn Arg Asn Arg Ala
        410                 415                 420

atc tca agc agt gct att tcg aga gct gtg tct gat gcc agt gct ggt      1351
Ile Ser Ser Ser Ala Ile Ser Arg Ala Val Ser Asp Ala Ser Ala Gly
    425                 430                 435

gat tat ggg agt gct att gag aca ctg gta act gca att tct tta att      1399
Asp Tyr Gly Ser Ala Ile Glu Thr Leu Val Thr Ala Ile Ser Leu Ile
440                 445                 450                 455

aaa caa tcc aaa gta tct gct gat gat cgt tgc aaa gtt ctt att agt      1447
Lys Gln Ser Lys Val Ser Ala Asp Asp Arg Cys Lys Val Leu Ile Ser
                460                 465                 470

tct ttg caa gat tgc ctt cat gga att gag tcc aag tct tat ggt tct      1495
Ser Leu Gln Asp Cys Leu His Gly Ile Glu Ser Lys Ser Tyr Gly Ser
            475                 480                 485

gga tca aga cgt gaa cga tca aga gag agg gac cat agt aga tca cga      1543
Gly Ser Arg Arg Glu Arg Ser Arg Glu Arg Asp His Ser Arg Ser Arg
        490                 495                 500

gaa aag agt cga cgt cat aaa tcc cgt agt aga gac cgt cat gac gat      1591
Glu Lys Ser Arg Arg His Lys Ser Arg Ser Arg Asp Arg His Asp Asp
    505                 510                 515

tat tac aga gag aga agc aga gaa cga gag agg cac cgg gat cgt gac      1639
Tyr Tyr Arg Glu Arg Ser Arg Glu Arg Glu Arg His Arg Asp Arg Asp
520                 525                 530                 535

cga gac cgt gac cga gag cgt gac cga gag cgc gaa tat cgt cat cgt      1687
Arg Asp Arg Asp Arg Glu Arg Asp Arg Glu Arg Glu Tyr Arg His Arg

                540                 545                 550

tagaagctga aggaagagga tcaccttcca agacaaaaca gtcttcatgg gccaaaaatg 1747

acgcttgtcc agcagtttgc ttcttgtgat tgaactgaac ctgtaaggat tcatggataa 1807

aatgaacagg aatagatctg aataaagcaa atctgcataa atggtaacca gtagctctac 1867

ttttattttt tatgttgctt aactgtttta tttgaaggaa acctgtgtga tttaaaaagt 1927

tatagctttt gcaactttat tactggttat atacatttgg ccattatgat gtgcaagcaa 1987

ttggaaaaaa agtcaagtaa atgcttgttt ttgtagtagt ttgttcttgt taaaaatgtt 2047

tatatgataa tgtctgtaaa cagcatcact ttgattacaa tagatgtagt gttgtaataa 2107

actgtttaat ggggctgatg tgtaaagctg ttcaagttat ttgatgttta cacctcaggg 2167

aaagtcttgt gttcagcaat atctaaagat aatgttacta tgacaacatt tttactgtcc 2227

tttaaagcat tgcaatagcg tttttggata tgcctcaatc taatcttgcg ttcagtgaat 2287

taaacatagt aattaagtgt cttttgccct tgattttgat attagaatag gtgattacat 2347

ggatatttaa tatttctata ttctgctttt ctagctgttt ttacctagtt agcttgtgac 2407

tttgctgaat ggtatgtaaa cttgtaaaaa tagagatttg acagacatag caatctagtc 2467

aatgtgtaag gggtcaaaaa aaacagaggt tttaacacat aagtaaaaac ccgtacatat 2527

ttgatgtgta atgcaggtta attacaacac agatgtaccg aaacacttaa ttgtgaaccg 2587

ctaacattga agaaattttg acaattccga tttgatgctg caattacttg ctgtttttat 2647

tgatcttatg gtttatttct taagccatag tcagtgtaaa tacagccctg cagcaggtaa 2707

atgtgagtaa agagagcctt atattttcca attggtataa aatttttgaa ggatgtgatg 2767

ttcattaaca ttcggttgta ttccccagta tttgtaatgg gaaattacag ataaaccgtg 2827

tctgcacagt ttaaggaata ctatgtatat tcatgcaccg tattgattca tgctatagtt 2887

acttaatcaa agattttttt caaacctgcc ttacatatag gcccacttta aaagcacctg 2947

actagcatgt gttcttgatt gcaaaattgg cagaggcagg gtgtcaactt gattaggtgt 3007

ttttatggga atgtaatttg aaatcactac ttcagaaatt tgacttaaaa ttcttgagca 3067

cgttaatatg tttttaagat ctgattatct ttgagagatc ttctgttaat acacattggt 3127

tgttaaagag tacccaaatt ctaggacaat gcttaaagtg ttaaaatacc ctagatactg 3187

tgttatgtgc aactgtagaa accctccaga aatttccact gctgttcttc actttcatct 3247

tgtctgctat caaaccactt ctgacaaaat tagctgtttt gaattaccca tatcactgcc 3307

agttttattt taaaatattt tgtgtttgaa gtatctgtgc atgggatcgt tgatgtttat 3367

cagaactgtt cactttcaga aatgattttt taaagcattt tgttgaaatg cggttgctt 3426

<210>  4
<211>  551
<212>  PRT
<213>  Homo sapiens

<400>  4

Met Ala Asp Gly Val Asp His Ile Asn Ile Tyr Ala Asp Val Gly Glu
1               5                   10                  15

Glu Phe Asn Gln Glu Ala Glu Tyr Gly Gly His Asp Gln Ile Asp Leu
            20                  25                  30

Tyr Asp Asp Val Ile Ser Pro Ser Ala Asn Asn Gly Asp Ala Pro Glu
        35                  40                  45

Asp Arg Asp Tyr Met Asp Thr Leu Pro Pro Thr Val Gly Asp Asp Val
    50                  55                  60

Gly Lys Gly Ala Ala Pro Asn Val Val Tyr Thr Tyr Thr Gly Lys Arg
65                  70                  75                  80

Ile Ala Leu Tyr Ile Gly Asn Leu Thr Trp Trp Thr Thr Asp Glu Asp
                85                  90                  95

Leu Thr Glu Ala Val His Ser Leu Gly Val Asn Asp Ile Leu Glu Ile
            100                 105                 110

Lys Phe Phe Glu Asn Arg Ala Asn Gly Gln Ser Lys Gly Phe Ala Leu
        115                 120                 125

Val Gly Val Gly Ser Glu Ala Ser Ser Lys Lys Leu Met Asp Leu Leu
    130                 135                 140

Pro Lys Arg Glu Leu His Gly Gln Asn Pro Val Val Thr Pro Cys Asn
145                 150                 155                 160

Lys Gln Phe Leu Ser Gln Phe Glu Met Gln Ser Arg Lys Thr Thr Gln
                165                 170                 175

Ser Gly Gln Met Ser Gly Glu Gly Lys Ala Gly Pro Pro Gly Gly Ser
            180                 185                 190

Ser Arg Ala Ala Phe Pro Gln Gly Gly Arg Gly Arg Gly Arg Phe Pro
        195                 200                 205

Gly Ala Val Pro Gly Gly Asp Arg Phe Pro Gly Pro Ala Gly Pro Gly
    210                 215                 220

Gly Pro Pro Pro Pro Phe Pro Ala Gly Gln Thr Pro Pro Arg Pro Pro
225                 230                 235                 240

Leu Gly Pro Pro Gly Pro Pro Gly Pro Pro Gly Pro Pro Pro Pro Gly
                245                 250                 255

Gln Val Leu Pro Pro Pro Leu Ala Gly Pro Pro Asn Arg Gly Asp Arg
            260                 265                 270

Pro Pro Pro Pro Val Leu Phe Pro Gly Gln Pro Phe Gly Gln Pro Pro
        275                 280                 285

Leu Gly Pro Leu Pro Pro Gly Pro Pro Pro Pro Val Pro Gly Tyr Gly
    290                 295                 300

Pro Pro Pro Gly Pro Pro Pro Pro Gln Gln Gly Pro Pro Pro Pro Pro
305                 310                 315                 320

Gly Pro Phe Pro Pro Arg Pro Pro Gly Pro Leu Gly Pro Pro Leu Thr
                325                 330                 335

Leu Ala Pro Pro Pro His Leu Pro Gly Pro Pro Pro Gly Ala Pro Pro
            340                 345                 350

Pro Ala Pro His Val Asn Pro Ala Phe Phe Pro Pro Pro Thr Asn Ser
        355                 360                 365

Gly Met Pro Thr Ser Asp Ser Arg Gly Pro Pro Pro Thr Asp Pro Tyr
    370                 375                 380

Gly Arg Pro Pro Pro Tyr Asp Arg Gly Asp Tyr Gly Pro Pro Gly Arg
385                 390                 395                 400

Glu Met Asp Thr Ala Arg Thr Pro Leu Ser Glu Ala Glu Phe Glu Glu
                405                 410                 415

Ile Met Asn Arg Asn Arg Ala Ile Ser Ser Ser Ala Ile Ser Arg Ala
            420                 425                 430

Val Ser Asp Ala Ser Ala Gly Asp Tyr Gly Ser Ala Ile Glu Thr Leu
        435                 440                 445

Val Thr Ala Ile Ser Leu Ile Lys Gln Ser Lys Val Ser Ala Asp Asp
    450                 455                 460

Arg Cys Lys Val Leu Ile Ser Ser Leu Gln Asp Cys Leu His Gly Ile
465                 470                 475                 480

Glu Ser Lys Ser Tyr Gly Ser Gly Ser Arg Arg Glu Arg Ser Arg Glu
                485                 490                 495

Arg Asp His Ser Arg Ser Arg Glu Lys Ser Arg Arg His Lys Ser Arg
            500                 505                 510

Ser Arg Asp Arg His Asp Asp Tyr Tyr Arg Glu Arg Ser Arg Glu Arg
        515                 520                 525

Glu Arg His Arg Asp Arg Asp Arg Asp Arg Asp Arg Glu Arg Asp Arg
    530                 535                 540

Glu Arg Glu Tyr Arg His Arg
545                 550

<210>  5
<211>  21
<212>  DNA
<213>  Homo sapiens

<400>  5
ccgtatattc ctgcacatat a                                                21

<210>  6
<211>  21
<212>  DNA/RNA
<213>  Artificial

<220>
<223>  Sense strand of siRNA CPSF5-1

<400>  6
guauauuccu gcacauauat t                                                21

<210>  7
<211>  21
<212>  DNA/RNA
<213>  Artificial

<220>
<223>  Antisense strand of siRNA CPSF5-1

<400>  7
uauaugugca ggaauauacg g                                                21

<210>  8
<211>  21
<212>  DNA

<213>  Homo sapiens

<400>  8
tagatgtagt gttgtaataa a                                                21


<210>  9
<211>  21
<212>  DNA/RNA
<213>  Artificial

<220>
<223>  Sense strand of siRNA CPSF6-1

<400>  9
gauguagugu uguaauaaat t                                                21


<210>  10
<211>  21
<212>  DNA/RNA
<213>  Artificial

<220>
<223>  Antisense strand of siRNA CPSF6-1

<400>  10
uuuauuacaa cacuacauct a                                                21


<210>  11
<211>  21
<212>  DNA
<213>  Homo sapiens

<400>  11
ctggttcagc ttcaagagaa a                                                21


<210>  12
<211>  21
<212>  DNA/RNA
<213>  Artificial

<220>
<223>  Sense strand of siRNA CPSF5-2

<400>  12
gguucagcuu caagagaaat t                                                21


<210>  13
<211>  21
<212>  DNA/RNA
<213>  Artificial

<220>
<223>  Antisense strand of siRNA CPSF5-2

<400>  13
uuucucuuga aggugaacca g                                                21

<210>  14
<211>  21
<212>  DNA
<213>  Homo sapiens

<400>  14
cgggaggaat ttgataagat t                                                21


<210>  15
<211>  21
<212>  DNA/RNA
<213>  Artificial

<220>
<223>  Sense strand of siRNA CPSF5-3

<400>  15
ggaggaauuu gauaagauut t                                                21


<210>  16
<211>  21
<212>  DNA/RNA
<213>  Artificial

<220>
<223>  Antisense strand of siRNA CPSF5-3

<400>  16
aaucuuauca aauuccuccc g                                                21


<210>  17
<211>  21
<212>  DNA
<213>  Homo sapiens

<400>  17
ccaggagaag atgaagttga a                                                21


<210>  18
<211>  21
<212>  DNA/RNA
<213>  Artificial

<220>
<223>  Sense strand of siRNA CPSF5-4

<400>  18
aggagaagau gaaguugaat t                                                21


<210>  19
<211>  21
<212>  DNA/RNA
<213>  Artificial

<220>
<223>  Antisense strand of siRNA CPSF5-4

<400>  19
uucaacuuca ucuucuccug g                                                21


<210>  20
<211>  21
<212>  DNA
<213>  Homo sapiens

<400>  20
cacggtcaga atcctgttgt a                                                21


<210>  21
<211>  21
<212>  DNA/RNA
<213>  Artificial

<220>
<223>  Sense strand of siRNA CPSF6-2

<400>  21
cggucagaau ccuguuguat t                                                21


<210>  22
<211>  21
<212>  DNA/RNA
<213>  Artificial

<220>
<223>  Antisense strand of siRNA CPSF6-2

<400>  22
uacaacagga uucugaccgt g                                                21


<210>  23
<211>  21
<212>  DNA
<213>  Homo sapiens

<400>  23
atcgggcaaa tggacaatca a                                                21


<210>  24
<211>  21
<212>  DNA/RNA
<213>  Artificial

<220>
<223>  Sense strand of siRNA CPSF6-3

<400>  24
cgggcaaaug gacaaucaat t                                                21

<210>  25
<211>  21
<212>  DNA/RNA
<213>  Artificial

<220>
<223>  Antisense strand of siRNA CPSF6-3

<400>  25
uugauugucc auuugcccga t                                                21


<210>  26
<211>  21
<212>  DNA
<213>  Homo sapiens

<400>  26
aacgtgcaat atgcaaataa t                                                21


<210>  27
<211>  21
<212>  DNA/RNA
<213>  Artificial

<220>
<223>  Sense strand of siRNA CPSF6-4

<400>  27
cgugcaauau gcaaauaaut t                                                21


<210>  28
<211>  21
<212>  DNA/RNA
<213>  Artificial

<220>
<223>  Antisense strand of siRNA CPSF6-4

<400>  28
auuauuugca uauugcacgt t                                                21


<210>  29
<211>  23
<212>  DNA
<213>  Artificial

<220>
<223>  Sense primer for amplifying CPSF5 mRNA

<400>  29
accgttgttt gaactgtacg aca                                              23


<210>  30
<211>  19
<212>  DNA
<213>  Artificial

<220>
<223>  Antisense primer for amplifying CPSF5 mRNA

<400>  30
cctgctcagc agctgagga                                                    19


<210>  31
<211>  28
<212>  DNA
<213>  Artificial

<220>
<223>  Probe for detecting CPSF5 mRNA

<400>  31
tccgggatac ggacccatca tttctagt                                          28


<210>  32
<211>  22
<212>  DNA
<213>  Artificial

<220>
<223>  Sense primer for amplifying CPSF6 mRNA

<400>  32
agcttgtgat tttgctgaat gg                                                22


<210>  33
<211>  24
<212>  DNA
<213>  Artificial

<220>
<223>  Antisense primer for amplifying CPSF6 mRNA

<400>  33
ttttttgacc cctaacacat tgaa                                              24


<210>  34
<211>  38
<212>  DNA
<213>  Artificial

<220>
<223>  Probe for detecting CPSF6 mRNA

<400>  34
atgtaaacgt gtaaaaactg aaatctgaca gagcaatc                              38


<210>  35
<211>  19
<212>  DNA
<213>  Artificial

<220>
<223>  Sense primer for amplifying beta-actin mRNA

<400>  35
tcctggcctc actgtccac                                                    19


<210>  36
<211>  19
<212>  DNA
<213>  Artificial

<220>
<223>  Antisense primer for amplifying beta-actin mRNA

<400>  36
gggccggact catcgtact                                                    19


<210>  37
<211>  26
<212>  DNA
<213>  Artificial

<220>
<223>  Probe for detecting beta-actin mRNA

<400>  37
ttccagcaga tgtggatcag caagca                                            26